# EUROPÄISCHE PATENTANMELDUNG

(11) **EP 4 245 837 A1**
(43) Veröffentlichungstag der Anmeldung: **20.09.2023**
(21) Anmeldenummer: 23184022.4
(22) Anmeldetag: 06.06.2019
(51) Int. Cl.: C12M 1/00, B01D 29/56, C12M 1/34, C12M 3/00

(54) **MODULARES PROZESSIERSYSTEM UND VERFAHREN ZUM MODULAREN AUFBAU EINES PROZESSIERSYSTEMS**

(30) Priorität: 19.06.2018 DE 102018004909
(62) Teilanmeldung aus: 19731168.1
(71) Anmelder: Sartorius Stedim Biotech GmbH, 37079 Göttingen (DE)
(72) Erfinder: LEUTHOLD, Martin, Göttingen (DE); WEISSHAAR, Stefan, Adelebsen (DE)
(74) Vertreter: Müller-Boré & Partner Patentanwälte PartG mbB

(57) **Zusammenfassung**

Die vorliegende Erfindung betrifft ein modulares Prozessiersystem für biopharmazeutische Prozesse, das umfasst: zumindest eine erste und zweite Prozessiereinheit, welche fluidisch miteinander verbindbar sind; zumindest eine Adapterplatte, welche von zumindest einem Fluidstrom durchströmbar ist, welcher von der ersten Prozessiereinheit zu der zweiten Prozessiereinheit strömt. Die Adapterplatte weist einen Adapterkanal auf, durch den der Fluidstrom strömen kann, wobei der Fluidstrom über zumindest eine Zutrittsöffnung in den Adapterkanal einströmbar ist und über zumindest eine Austrittsöffnung ausströmbar ist. Der Adapterkanal ist mit zumindest einem Hilfszu- und/oder Hilfsausgang ausgebildet ist. In dem Adapterkanal ist zumindest ein Ventil angeordnet, welches dazu ausgelegt ist, den Fluidstrom zwischen der ersten Prozessiereinheit und der zweiten Prozessiereinheit zumindest teilweise umzulenken.

## Beschreibung

Die vorliegende Erfindung betrifft ein modulares Prozessiersystem für biopharmazeutische Prozesse und ein Verfahren zum modularen Aufbau eines Prozessiersystems für biopharmazeutische Prozesse.

Aus der Biopharmazie sind Prozesse wie beispielsweise Zellabtrennung (beispielsweise durch Tiefenfiltration), Sterilfiltration, Chromatographieschritte, Virusinaktivierung, Virusfiltration und/oder Crossflow-Filtration bekannt. Alle diese Prozesse stellen Grundoperationen dar, die regelmäßig zu unterschiedlichen Gesamtprozessen verschaltet werden. Im Stand der Technik werden beispielsweise bezüglich Filtrationsprozessen hierzu unterschiedliche Filtersysteme bzw. Filteranlagen, die jeweils für die Aufarbeitung der unterschiedlichen Prozessschritte zuständig sind, miteinander verknüpft. Dabei ist eine Kommunikation der verschiedenen Filtersysteme notwendig, um Produktströme und Stellgrößen im Prozess entsprechend abzugleichen. Weiterhin werden im Stand der Technik lediglich Standard-Filtereinheiten verwendet, vgl. z.B. DE 34 41 249 C2.

Es ist somit die Aufgabe der Erfindung, ein Prozessiersystem für biopharmazeutische Prozesse bereitzustellen, das eine vereinfachte und kompaktere Verschaltung von Prozessschritten ermöglicht.

Diese Aufgabe wird gemäß einem ersten Aspekt der Erfindung durch ein modulares Prozessiersystem für biopharmazeutische Prozesse gelöst, welches umfasst:
- zumindest eine erste und zweite Prozessiereinheit, welche fluidisch miteinander verbindbar sind;
- zumindest eine Adapterplatte, welche von zumindest einem Fluidstrom durchströmbar ist, welcher von der ersten Prozessiereinheit zu der zweiten Prozessiereinheit strömt;
wobei die Adapterplatte derart ausgebildet ist, dass der Fluidstrom zwischen der ersten Prozessiereinheit und der zweiten Prozessiereinheit mit Hilfe der Adapterplatte zumindest teilweise umlenkbar ist und/oder der Fluidstrom, bevorzugt dessen Druck, mit Hilfe der Adapterplatte regel- oder steuerbar ist.

Als "Prozessiereinheit" wird insbesondere eine Einheit verstanden, innerhalb der ein spezieller Prozessschritt für das gewünschte Verfahren ausgeführt wird. Insbesondere erfolgt innerhalb einer Prozessiereinheit eine Trennung von Komponenten eines Fluidstroms. Beispielsweise kann eine Prozessiereinheit eine Einheit zur Zellabtrennung (beispielsweise durch Tiefenfiltration), zur Sterilfiltration, für einen Chromatographieschritt, zur Virusinaktivierung oder zur Crossflow-Filtration sein. Die Prozessiereinheit kann hier wie auch im Stand der Technik als Standard-Prozessiereinheit mit den gewünschten Spezifikationen ausgewählt werden. Die erste und zweite Prozessiereinheit können identisch ausgebildet sein oder zumindest teilweise unterschiedliche Eigenschaften aufweisen. Beispielsweise können die erste und zweite Prozessiereinheit in ihrer Größe variieren, können jedoch mit Hilfe der Adapterplatte dennoch in einfacher Weise miteinander verschaltet bzw. kombiniert werden. Vorzugsweise werden in einem Prozessiersystem unterschiedliche Prozessiereinheiten miteinander verschaltet, die beispielsweise unterschiedliche Trennmedien verwenden oder für unterschiedliche Prozessierstufen eines Verfahrens zuständig sind. Gleiche Prozessiereinheiten können dann zum Einsatz kommen, wenn eine Kapazitätserweiterung gewünscht wird.

Die Adapterplatte ist bezüglich des Fluidstroms zwischen der ersten Prozessiereinheit und der zweiten Prozessiereinheit geschaltet und verbindet somit die erste Prozessiereinheit und die zweite Prozessiereinheit fluidisch.

Durch die Adapterplatte, die (zumindest teilweise) zwischen zwei Prozessiereinheiten in einem modularen Prozessiersystem schaltbar ist, können erforderliche Anpassungen bezüglich des Fluidstroms vorgenommen werden, die es ermöglichen, Prozessiereinheiten (insbesondere Standard-Prozessiereinheiten) miteinander fluidisch zu verbinden. Dabei kann der Fluidstrom entsprechend zumindest teilweise umgelenkt bzw. abgelenkt bzw. in der Flußrichtung geändert werden und/oder der Fluidstrom mit Hilfe der Adapterplatte, bevorzugt bzw. insbesondere dessen Druck, mit dem das Fluid auf die zweite Prozessiereinheit trifft, geregelt oder gesteuert werden. Es können jedoch auch beide Anpassungen gleichzeitig innerhalb einer Adapterplatte vorgenommen werden.

Somit kann zumindest eine Adapterplatte zwischen zwei aufeinander folgenden Prozessiereinheiten in einem Prozessiersystem angeordnet werden, um die nötigen Anpassungen bezüglich des Fluidstroms vornehmen zu können.

Eine Verschaltung von Prozessschritten basierend auf einem modular aufgebauten Prozessiersystem mit mindestens einer Adapterplatte zwischen zwei Prozessiereinheiten ermöglicht die Kombination von jeglichen Prozessschritten in einem Prozessiersystem, so dass der Bedarf an Stellfläche und Anlagenkomponenten reduziert werden kann. Entsprechend verringern sich die notwendigen Investitionen. Weiterhin wird insbesondere eine kompakte und/oder flexible Verschaltung für die kontinuierliche Betriebsweise in einem Prozessiersystem, inklusive der Überwachung mehrerer Grundoperationen, ermöglicht.

Vorzugsweise umfasst das Prozessiersystem mehrere parallel verschaltete erste Prozessiereinheiten und/oder mehrere parallel verschaltete zweite Prozessiereinheiten, wobei die ersten Prozessiereinheiten und die zweiten Prozessiereinheiten jeweils eine Prozessiereinheitengruppe bilden.

Mit anderen Worten werden mehrere in Folge verschaltete Prozessiereinheiten, die ohne Adapterplatte verschaltet sind, als Prozessiereinheitengruppe bezeichnet. Innerhalb einer Prozessiereinheitengruppe sind die Prozessiereinheiten vorzugsweise parallel verschaltet. Mittels einer erfindungsgemäßen Adapterplatte können somit eine Prozessiereinheitengruppe mit einer Prozessiereinheit oder zwei Prozessiereinheitengruppen miteinander verschaltet werden.

Vorzugsweise weist die Adapterplatte einen Adapterkanal auf, durch den der Fluidstrom strömen kann, wobei der Fluidstrom über zumindest eine Zutrittsöffnung in den Adapterkanal einströmbar ist und über zumindest eine Austrittsöffnung ausströmbar ist.

Als "Adapterkanal" wird insbesondere ein Kanal durch die bzw. in der Adapterplatte verstanden, durch den der Fluidstrom, der von der ersten Prozessiereinheit zu der zweiten Prozessiereinheit strömt, strömen kann. Vorzugsweise ist der Adapterkanal ein Umlenkkanal.

Vorzugsweise weist der Adapterkanal eine Vorrichtung zur Entlüftung auf und/oder benötigt keine Dichtungen, wie dies üblicherweise in Prozessiereinheiten von Nöten ist.

Weiterhin ist es bevorzugt, dass die zumindest eine Zutrittsöffnung derart angeordnet ist, dass diese mit einem Ablaufkanal der ersten Prozessiereinheit verbindbar ist und/oder
wobei die zumindest eine Austrittsöffnung derart angeordnet ist, dass diese mit einem Zulaufkanal der zweiten Prozessiereinheit verbindbar ist.

Als "Ablaufkanal" der ersten Prozessiereinheit wird insbesondere der Kanal verstanden, über den ein Fluidstrom aus der ersten Prozessiereinheit austreten kann. Weist das Prozessiersystem mehrere erste Prozessiereinheiten auf, handelt es sich bei dem Ablaufkanal, der mit dem Adapterkanal gekoppelt ist, um den Ablaufkanal der ersten Prozessiereinheit, die direkt vor der Adapterplatte angeordnet ist. Handelt es sich bei der ersten Prozessiereinheit beispielsweise um eine Filtereinheit, kann es sich bei dem Fluid, das über den Ablaufkanal aus der ersten Prozessiereinheit austritt, um ein Filtrat handeln, also das durch die erste Prozessiereinheit filtrierte Fluid. Handelt es sich bei der ersten Prozessiereinheit um eine Crossflowfiltrationseinheit, kann das Fluid, das über den Ablaufkanal aus der ersten Prozessiereinheit austritt, auch Retentat sein (also ein nicht filtrierter Anteil des Fluidstroms). Im Rahmen der Crossflowfiltration strömt somit entweder das Filtrat oder das Retentat von der ersten Prozessiereinheit über die Adapterplatte zu der zweiten Prozessiereinheit, um dort weiter prozessiert zu werden.

Als "Zulaufkanal" der zweiten Prozessiereinheit wird insbesondere der Kanal verstanden, über den der Fluidstrom aus der Adapterplatte in die zweite Prozessiereinheit strömt. Weist das Prozessiersystem mehrere zweite Prozessiereinheiten auf, handelt es sich bei dem Zulaufkanal, der mit dem Adapterkanal gekoppelt ist, um den Zulaufkanal der zweiten Prozessiereinheit, die der Adapterplatte direkt nachgeschaltet ist. Handelt es sich bei der zweiten Prozessiereinheit um eine Filtereinheit, entspricht dieser Fluidstrom der Speiseflüssigkeit, die durch die zweite Prozessiereinheit zu filtrieren ist.

Aufgrund einer derartigen Verschaltung der ersten Prozessiereinheit mit der zweiten Prozessiereinheit können zwei Prozessiereinheiten in kompakter Weise seriell miteinander verschaltet werden.

Vorzugsweise weist die Adapterplatte zumindest zwei Zutrittsöffnungen und zumindest zwei Austrittsöffnungen auf,
wobei zumindest eine erste Zutrittsöffnung derart angeordnet ist, dass der Fluidstrom aus einem Ablaufkanal der ersten Prozessiereinheit in den Adapterkanal einströmbar ist,
wobei zumindest eine zweite Zutrittsöffnung derart angeordnet ist, dass der Fluidstrom aus einem Zulaufkanal der ersten Prozessiereinheit in den Adapterkanal einströmbar ist,
wobei zumindest eine erste Austrittsöffnung derart angeordnet ist, dass der Fluidstrom aus der ersten Austrittsöffnung in einen Ablaufkanal der zweiten Prozessiereinheit einströmbar ist, und
wobei zumindest eine zweite Austrittsöffnung derart angeordnet ist, dass der Fluidstrom aus der zweiten Austrittsöffnung in einen Zulaufkanal der zweiten Prozessiereinheit einströmbar ist.

Weiterhin ist es bevorzugt, dass der Adapterkanal umfasst:
- einen ersten Kanalbereich, der zwischen der ersten Zutrittsöffnung und der ersten Austrittsöffnung angeordnet ist;
- einen zweiten Kanalbereich, der zwischen der zweiten Zutrittsöffnung und der zweiten Austrittsöffnung angeordnet ist; und
- einen Verbindungskanalbereich, der den ersten und zweiten Kanalbereich fluidisch verbindet.

Vorzugsweise ist in dem Adapterkanal zumindest ein Umlenkelement derart verschiebbar angeordnet ist, dass ein Fluidstrom durch die Adapterplatte derart umgelenkt werden kann, dass entweder:
- ein erster Fluidstrom über die erste Zutrittsöffnung in den Adapterkanal einströmen kann und über die erste Austrittsöffnung ausströmen kann und ein zweiter Fluidstrom über die zweite Zutrittsöffnung in den Adapterkanal einströmen kann und über die zweite Austrittsöffnung aus dem Adapterkanal ausströmen kann;
oder:
- ein Fluidstrom über die erste Zutrittsöffnung in den Adapterkanal einströmen kann und über die zweite Austrittsöffnung aus dem Adapterkanal ausströmen kann.

Vorzugsweise ist das Umlenkelement derart angeordnet, dass das Umlenkelement in den Verbindungskanalbereich hineinschiebbar ist, um einen Fluidstrom durch den Verbindungskanalbereich zu blockieren. Soll einen Fluidstrom durch den Verbindungskanalbereich ermöglicht werden, ist das Umlenkelement zumindest teilweise aus dem Verbindungskanalbereich herausschiebbar. Vorzugsweise verschließt das Umlenkelement den Verbindungskanalbereich fluiddicht. Eine mögliche Ausführung des Umlenkelements ist eine verschiebbare Verschlussplatte. Vorzugsweise ist das Umlenkelement händisch und/oder automatisch über eine externe Steuerung verschiebbar. Verschließt das Umlenkelement den Verbindungskanalbereich, sind beide Prozessiereinheiten parallel verschaltet.

Weiterhin kann jeweils ein Umlenkelement in dem ersten Kanalbereich und/oder dem zweiten Kanalbereich angeordnet sein. Dieses wird so in den entsprechenden Kanalbereich eingeschoben, dass durch das jeweilige Umlenkelement die erste Austrittsöffnung und/oder die zweite Zutrittsöffnung blockierbar sind. Sind die genannten Öffnungen durch die Umlenkelemente blockiert, ist die erste und zweite Filtereinheit seriell verschaltet. Das Umlenkelement in dem Verbindungsbereich ist hier hingegen in einer rückgezogenen Position, so dass ein Fluidstrom durch den Verbindungskanalbereich möglich ist.

Bevorzugt ist die Verschiebung der Umlenkelemente miteinander gekoppelt. Öffnet man beispielsweise das Umlenkelement in dem Verbindungskanalbereich, schließen sich die Umlenkelemente in dem ersten und zweiten Kanalbereich und umgekehrt.

Vorzugsweise umfasst das modulare Prozessiersystem zwei Umlenkelemente, welche als Mehrwegventil ausgebildet sind, wobei ein erstes Ventil in dem ersten Kanalbereich angeordnet ist und ein zweites Ventil in dem zweiten Kanalbereich angeordnet ist, wobei die Ventile derart ausgelegt sind, dass entweder:
- ein erster Fluidstrom über die erste Zutrittsöffnung in den Adapterkanal einströmen kann und über die erste Austrittsöffnung ausströmen kann und ein zweiter Fluidstrom über die zweite Zutrittsöffnung in den Adapterkanal einströmen kann und über die zweite Austrittsöffnung aus dem Adapterkanal ausströmen kann;
oder:
- ein Fluidstrom über die erste Zutrittsöffnung in den Adapterkanal einströmen kann und über die zweite Austrittsöffnung aus dem Adapterkanal ausströmen kann.

Mit Hilfe der Ventile wird eine alternative Variante zu dem verschiebbaren Umlenkelement bereitgestellt, die es ebenfalls in einfacher und kompakter Weise ermöglicht, die erste und zweite Prozessiereinheit seriell oder parallel zu verschalten. Die Ventile sind vorzugsweise durch eine externe Steuereinheit gesteuert. Die beiden Ventile können separat oder zusammen steuert werden.

In einer bevorzugten Ausführungsform umfasst das Mehrwegventil ein Ventilrohr, welches jeweils verlagerbar (insbesondere rotierbar) in dem ersten und zweiten Kanalbereich angeordnet ist und in entsprechender Weise von dem ersten und zweiten Fluidstrom durchströmbar ist,
wobei in einer Mantelfläche des Ventilrohrs zumindest zwei Ventilöffnungen angeordnet sind, die in Verlagerungsrichtung (insbesondere Rotationsrichtung) voneinander versetzt angeordnet sind, so dass in Abhängigkeit der Verlagerungsstellung (insbesondere Rotationsstellung) des Ventilrohrs in dem jeweiligen Kanalbereich ein Fluidstrom durch den Adapterkanal umlenkbar ist.

Das Ventilrohr erstreckt sich jeweils zumindest teilweise in dem ersten und zweiten Kanalbereich und ist dort verlagerbar, insbesondere drehbar bzw. rotierbar gelagert. Eine Verlagerung (insbesondere Rotation) kann händisch und/oder durch ein externes Steuergerät erfolgen. In der Mantelfläche des Ventilrohrs sind zumindest zwei Ventilöffnungen ausgebildet. Diese sind in Bezug auf die Verlagerungsrichtung (insbesondere Rotationsrichtung) derart voneinander versetzt angeordnet, dass in einer ersten Stellung des Ventilrohrs ein erster Fluidstrom bzw. ein zweiter Fluidstrom ermöglicht wird. Das heißt, dass beispielsweise der erste Fluidstrom über die erste Zutrittsöffnung der Adapterplatte durch eine erste Ventilöffnung die zumindest teilweise mit der ersten Zutrittsöffnung überlappt, in das Ventilrohr eintritt. Die zweite Ventilöffnung überlappt zumindest teilweise die erste Austrittsöffnung, so dass der erste Fluidstrom über die erste Austrittsöffnung aus der Adapterplatte strömen kann. Dies gilt in entsprechender Weise für den zweiten Fluidstrom. Ein Fluidstrom durch den Verbindungskanalbereich wird aufgrund der Stellung der Ventilöffnungen in den jeweiligen Kanalbereichen blockiert, da keine Ventilöffnung mit dem Verbindungskanalbereich überlappt. Da der erste und zweite Fluidstrom durch die Mehrwegventile jedoch gestattet wird, sind die erste und zweite Filtereinheit parallel miteinander verschaltet.

Vorzugsweise weisen die Ventilrohre jedoch zumindest drei Ventilöffnungen auf. Diese sind derart angeordnet, dass in einer ersten Verlagerungsstellung (insbesondere Rotationsstellung) des Ventilrohrs zumindest zwei Ventilöffnungen derart ausgerichtet sind, dass die oben beschriebenen ersten und zweiten Fluidströme gestattet werden und so die erste und zweite Prozessiereinheit parallel verschaltet sind. Ein Fluidstrom durch den Verbindungskanalbereich wird hier jedoch blockiert, da keine Ventilöffnung mit dem Verbindungskanalbereich überlappt.

Aufgrund der Anordnung der zumindest drei Ventilöffnungen sind in einer zweiten Verlagerungsstellung (insbesondere Rotationsstellung) des jeweiligen Ventilrohrs jedoch entweder der Durchgang zu einer Zutrittsöffnung oder zu einer Austrittsöffnung blockiert, während der Zugang zu dem Verbindungskanalbereich gestattet wird. Dies ermöglicht insbesondere eine serielle Verschaltung der ersten und zweiten Prozessiereinheit.

Mit anderen Worten sind in einer seriellen Verschaltung die Mehrwegventile derart auszurichten, dass ein Fluidstrom über die erste Zutrittsöffnung der Adapterplatte und über eine Ventilöffnung, die zumindest teilweise mit der ersten Zutrittsöffnung überlappt, in das erste Ventilrohr eintreten kann. Über eine Ventilöffnung, die zumindest teilweise mit dem Verbindungskanalbereich überlappt, kann der Fluidstrom in den Verbindungskanalbereich strömen. Der Durchgang zu der ersten Austrittsöffnung wird durch das Ventilrohr blockiert, da keine Ventilöffnung mit der ersten Austrittsöffnung überlappt. Anschließend kann der Fluidstrom über eine Ventilöffnung des zweiten Ventilrohrs, die zumindest teilweise mit dem Verbindungskanalbereich überlappt, in das zweite Ventilrohr einströmen. Über eine Ventilöffnung in dem zweiten Ventilrohr, die zumindest teilweise mit der zweiten Austrittsöffnung der Adapterplatte überlappt, kann der Fluidstrom aus der Adapterplatte ausströmen. Der Durchgang zu der zweiten Zutrittsöffnung wird durch das Ventilrohr blockiert, da keine Ventilöffnung mit der zweiten Zutrittsöffnung überlappt.

Mit anderen Worten wirkt das Ventilrohr als ein Absperrhahn, der bestimmte Durchgangswege durch die Adapterplatte je nach Bedarf blockiert bzw. freigibt.

Vorzugsweise ist in bzw. an dem Adapterkanal zumindest ein Sensor angeordnet.

Der Sensor kann zur Messung des Drucks, des Volumenstroms, des UV-Werts, des pH-Werts, der Trübung und/oder der Viskosität des Fluids, das durch die Adapterplatte strömt, genutzt werden. Insbesondere können hierzu kostengünstige Einweg-Sensoren genutzt werden. Die gemessenen Werte können wiederum zum Regeln und/oder Steuern des Prozesses, z.B. des Filtrationsprozesses, herangezogen werden. Hierzu können die gemessenen Werte an ein externes Steuergerät übermittelt werden, um dort verarbeitet zu werden und anschließend entsprechende Maßnahmen vornehmen zu können.

Weiterhin ist es bevorzugt, dass der Adapterkanal mit zumindest einem Hilfszu-und/oder Hilfsausgang ausgebildet ist.

Mit Hilfe des zumindest einen Hilfszu- und/oder Hilfsausgangs kann der Fluidstrom, der durch die Adapterplatte strömen kann, zumindest teilweise aus der Adapterplatte ab- und/oder wieder zugeführt werden. Dieser abgezweigte Fluidstrom kann beispielsweise für eine externe Verarbeitung des Fluidstroms aus mindestens einem vorgelagerten Prozessierschritt oder Filtrationsschritts bei einer (kontinuierlichen) Virusinaktivierung genutzt werden (z.B. die Virusinaktivierung nach Chromatographieschritt Protein A. Weiterhin kann über einen Hilfszugang eine externe Pumpe zugeschaltet werden und/oder ein Integritätstest an unterschiedlichen Prozessiereinheiten innerhalb des Prozessiersystems vorgenommen werden. Darüber hinaus kann der Fluidstrom aus der ersten Prozessiereinheit über einen Hilfsausgang abgeführt werden, um außerhalb zwischenzeitlich gepuffert und/oder regeneriert werden zu können. Weiterhin kann über einen Hilfseingang eine erste Prozessiereinheit getrennt gereinigt werden. Das heißt, es kann beispielsweise die Waschlösung aus der ersten Prozessiereinheit über den Hilfsausgang in der Adapterplatte ausströmen ohne den folgenden Prozessierschritt bzw. die folgende Prozessiereinheit zu kontaminieren. Bei der Waschlösung kann es sich z.B. um eine Lauge oder einen Waschpuffer handeln. Weiterhin kann über einen Hilfszugang ein Diafiltrationsmedium zum Lösungsmittelaustausch zugeführt werden. Grundsätzlich ist über einen Hilfszugang jede andere Form von einer Medienzugabe möglich. Insbesondere kann über einen Hilfsausgang eine Elution/Waschen/Reinigen einer vor-oder nachgeschalteten Chromatographiefiltervorrichtung erfolgen. Weiterhin kann das Prozessiersystem über den Hilfsausgang entlüftet werden und/oder es können Proben entnommen werden.

Es können mehrere Hilfszu- und/oder Hilfsausgänge in einer Adapterplatte vorgesehen sein, die unterschiedliche oder dieselben Funktionen erfüllen.

Vorzugsweise ist in bzw. an dem Adapterkanal zumindest eine Pumpe angeordnet.

Eine in die Adapterplatte integrierte Pumpe kann beispielsweise eine hinreichende Prozessier-, insbesondere Filtrationsleistung in der zweiten bzw. nächsten Prozessiereinheit ermöglichen. Dies ist insbesondere bei einer Serienschaltung notwendig, um den Flusswiderstand der nächsten Prozessiereinheit/Filtereinheit zu überwinden.

Als Pumpen eignen sich hier auch Einweg-Ausführungen, die kostengünstig in der Herstellung sind und keine Reinigungs- bzw. Wartungskosten verursachen.

In bevorzugten Ausführungsformen ist die Pumpe als Peristaltikpumpe oder Kolbenpumpe ausgebildet.

Eine Peristaltikpumpe, auch Schlauchpumpe genannt, ist eine Verdrängerpumpe, bei der das zu fördernde Fluid durch äußere mechanische Verformung eines Schlauches durch diesen hindurchgedrückt wird. Der Schlauch ist außen an einem Gehäuse eines Pumpenkopfes abgestützt und ist von innen durch Rollen und/oder Gleitschuhe abklemmbar, die sich an einem Rotor drehen (radiales Wirksystem) bzw. über eine Nockenwelle bewegbar (lineares oder auch horizontales Wirksystem). Bei beiden Bauarten führt die Bewegung dazu, dass sich eine Abklemmstelle entlang des Schlauches bewegt und dadurch das zu fördernde Fluid vorantreibt.

Vorzugsweise ist die Peristaltikpumpe in dem Verbindungskanalbereich angeordnet.

Eine Kolbenpumpe ist ebenfalls eine Verdrängerpumpe bei der ein Verdränger (Kolben) eine Hub-Bewegung, also eine geradlinige (translatorische) Bewegung, ausführt. Die Kolbenpumpe weist einen Kolben auf, der in einem Zylinder läuft, kombiniert mit einem Zu- und einem Ablauf, die jeweils durch ein Ventil verschließbar sind.

Vorzugsweise ist die Kolbenpumpe in bzw. an dem Verbindungskanalbereich der Adapterplatte angeordnet. Ein Abschnitt in dem Verbindungskanalbereich ist in Flussrichtung durch ein Einlassventil und durch ein Auslassventil abgetrennt. Über das Einlassventil kann das Fluid in diesen Abschnitt strömen und über das Auslassventil entweichen. Der Zylinder, in dem der Kolben gelagert ist und in diesem eine translatorische Bewegung ausführen kann, ist derart angeordnet, dass der Zylinder an dem Verbindungskanalbereich angeordnet und mit diesem fluidisch verbunden ist. Insbesondere ist der Zylinder zwischen dem Einlass- und Auslassventil an dem Verbindungskanalbereich angeordnet.

In einem ersten Takt, beim Ansaugen, führt der Kolben eine Rückwärtsbewegung aus, also eine Bewegung weg von dem Verbindungskanalbereich. Das Einlassventil öffnet sich und das zu fördernde Fluid kann in den Verbindungskanalbereich bzw. den Zylinder strömen. In einem zweiten Takt, bei der Förderbewegung schließt sich das Einlassventil und der Kolben bewegt sich in Richtung zu dem Verbindungskanalbereich. Es öffnet sich das Auslassventil und das Fördermedium wird herausgedrückt.

Vorzugsweise ist in dem Adapterkanal zumindest ein Flussbegrenzer angeordnet.

In einigen Filtrationsprozessen ist eine Regulierung des Flusses und/oder Druckes vorteilhaft. Beispielsweise kann es notwendig sein, den Durchfluss zu der zweiten Prozessiereinheit/Filtereinheit zu blockieren oder zu reduzieren, um einen zu hohen Eingangsdruck zu vermeiden. Weiterhin kann durch den Flussbegrenzer der benötigte Transmembrandruck in einer vorgeschalteten Single-Pass Tangentialflussfiltrationseinheit eingestellt werden. Hierbei beschreibt "SinglePass" in der Crossflowfiltration die Betriebsweise, in der ein Fluidstrom im Gegensatz zu einer klassischen Betriebsweise ohne Rezirkulation verarbeitet wird.

Der zumindest eine Flussbegrenzer ist in dem Adapterkanal, vorzugsweise in dem Verbindungskanalbereich, angeordnet. Dieser kann als Ventil ausgebildet sein.

Gemäß einem weiteren Aspekt der Erfindung wird die zugrunde liegende Aufgabe durch ein Verfahren zum modularen Aufbau eines Prozessiersystems gelöst, welche die folgenden Schritte umfasst:
- Bereitstellen zumindest einer ersten und zweiten Prozessiereinheit;
- Bereitstellen zumindest einer Adapterplatte, welche dazu ausgelegt ist, die erste und zweite Prozessiereinheit fluidisch zu verbinden, so dass zumindest ein Fluidstrom von der ersten Prozessiereinheit zu der zweiten Prozessiereinheit durch die Adapterplatte strömen kann; und
- Verbinden der ersten und zweiten Prozessiereinheit mit der Adapterplatte,
wobei die Adapterplatte derart gewählt und/oder eingestellt wird, dass der Fluidstrom zwischen der ersten Prozessiereinheit und der zweiten Prozessiereinheit mit Hilfe der Adapterplatte in vordefinierter Weise zumindest teilweise umlenkbar ist und/oder der Fluidstrom, bevorzugt dessen Druck, mit Hilfe der Adapterplatte in vordefinierter Weise regel- oder steuerbar ist.

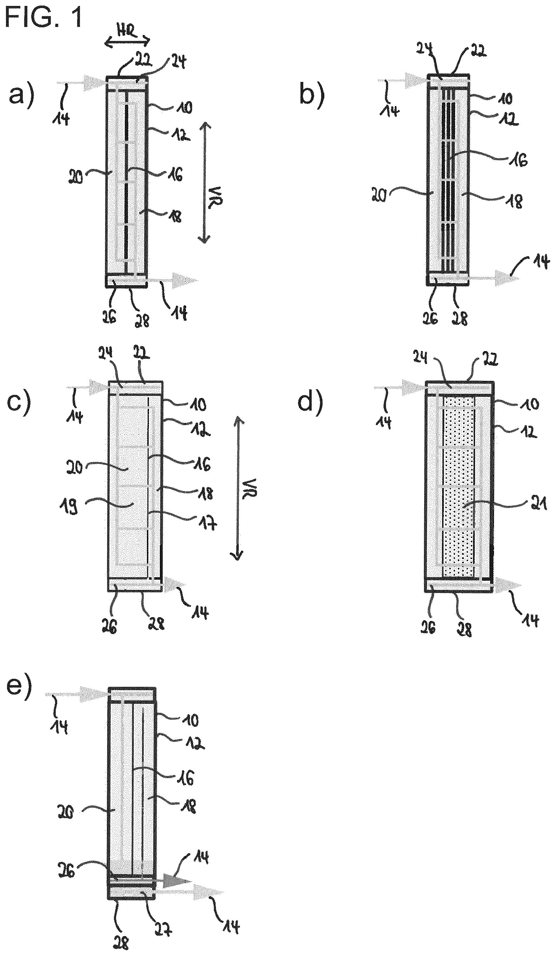

Diese und andere Ziele, Merkmale und Vorteile der vorliegenden Erfindung werden durch das Studium der folgenden detaillierten Beschreibung bevorzugter Ausführungsformen und der beigefügten Zeichnungen klarer. Weiterhin wird darauf hingewiesen, dass, obwohl Ausführungsformen separat beschrieben werden, einzelne Merkmale dieser Ausführungsformen zu zusätzlichen Ausführungsformen kombiniert werden können.
- Fig. 1 a)-e): zeigen einen grundlegenden Aufbau verschiedener Prozessiereinheiten für biopharmazeutische Prozesse;
- Fig. 2a): zeigt ein Prozessiersystem mit zwei Prozessiereinheitengruppen gemäß einer Ausführungsform, die mit Hilfe einer Adapterplatte parallel verschaltet sind;
- Fig. 2b): zeigt das Prozessiersystem aus Fig. 2a), in dem zwei Prozessiereinheitengruppen mit Hilfe der Adapterplatte seriell verschaltet sind;
- Fig. 2c): zeigt das Prozessiersystem aus Fig. 2b, in dem die einzelnen Prozessiereinheiten mittels Abschlusshalterungen zusammengehalten sind;
- Fig. 3a): zeigt eine Schnittansicht durch eine Adapterplatte aus den Figuren 2a) und 2b) mit einem Mehrwegventil;
- Fig. 3b): zeigt das Mehrwegventil aus Figur 3a);
- Fig. 4: zeigt ein Prozessiersystem mit einer Adapterplatte gemäß einer weiteren Ausführungsform, in der ein Sensor integriert ist;
- Fig. 5a): zeigt eine Schnittansicht einer zweiteiligen Adapterplatte mit einem Sensor gemäß einer weiteren Ausführungsform;
- Fig. 5b): zeigt eine perspektivische Ansicht der Adapterplatte aus Fig. 5a);
- Fig. 6a): zeigt eine Schnittansicht einer Adapterplatte gemäß einer weiteren Ausführungsform mit einer Hilfsabzweigung, in der ein Sensor integriert ist;
- Fig. 6b): zeigt eine perspektivische Ansicht der Adapterplatte aus Fig. 6a);
- Fig. 7a): zeigt ein Prozessiersystem mit einer Adapterplatte gemäß einer weiteren Ausführungsform, die zwei Hilfsaus- und -zugänge aufweist;
- Fig. 7b): zeigt das Prozessiersystem aus Fig. 7a) mit einem Hilfsausgang und einem Hilfsventil;
- Fig. 7c): zeigt das Prozessiersystem aus Fig. 7b) mit einem externen Mischtank;
- Fig. 8a): zeigt eine Schnittansicht durch eine zweiteilige Adapterplatte mit verschiebbaren Umlenkelementen, wobei der Verbindungskanalbereich durch das Umlenkelement blockiert ist;
- Fig. 8b): zeigt die Adapterplatte aus Fig. 8a), wobei ein Fluidstrom durch den Verbindungskanalbereich durch das Umlenkelement freigegeben ist;
- Fig. 9: zeigt ein Prozessiersystem mit einer Adapterplatte gemäß einer weiteren Ausführungsform, in der eine Pumpe integriert ist;
- Fig. 10a): zeigt eine Schnittansicht durch eine Adapterplatte mit einer Kolbenpumpe in einer Ansaugposition;
- Fig. 10b): zeigt eine Schnittansicht der Adapterplatte aus Figur 10a) in einer Hubposition;
- Fig. 11a): zeigt eine Explosionsansicht einer Adapterplatte mit einer Peristaltikpumpe gemäß einer weiteren Ausführungsform;
- Fig. 11b): zeigt eine Schnittansicht der Adapterplatte aus Fig. 11a);
- Fig. 12a): zeigt ein Prozessiersystem mit einer Adapterplatte gemäß einer weiteren Ausführungsform, in der ein Flussbegrenzer integriert ist;
- Fig. 12b): zeigt eine Schnittansicht der Adapterplatte aus Fig. 12a);
- Fig. 13: zeigt ein Filtersystem mit einer Adapterplatte für eine mögliche Filtererweiterung gemäß einer weiteren Ausführungsform;
- Fig. 14: zeigt eine Schnittansicht einer Adapterplatte gemäß einer weiteren Ausführungsform mit einer Berstdruckscheibe.
Für biopharmazeutische Prozesse bestehen verschiedene Prozessiereinheiten, die im Rahmen der vorliegenden Erfindung zum Einsatz kommen können. Die **Figuren 1a) bis 1e****)** zeigen einen grundlegenden Aufbau verschiedener Prozessiereinheiten, die im Rahmen der vorliegenden Erfindung verwendet werden können. Es handelt sich hierbei um eine Auswahl, jedoch keine abschließende Liste.

**Fig. 1a****)** zeigt einer Prozessiereinheit 10, die dazu verwendet werden kann, einen bestimmten Filtrationsschritt in einem biopharmazeutischen Prozess zu übernehmen. Hierzu weist die Prozessiereinheit 10 ein Prozessiergehäuse 12 auf, das von einem Fluidstrom 14 durchströmbar ist. Der Fluidstrom 14 umfasst das zu filtrierende Medium. In dem Prozessiergehäuse 12 ist zumindest ein Filtermedium 16 angeordnet, das ein poröses Material umfasst, das danach gewählt bzw. verwendet wird, welche Partikel bzw. welche Stoffe mit Hilfe der Prozessiereinheit 10 aus dem Fluidstrom 14 herausgefiltert werden soll. Beispielsweise kann es sich bei dem Filtermedium 16 um einen Virusfilter, einen Sterilfilter, einen Tiefenfilter oder einen Membranadsorber handeln. Das Filtermedium 16 ist vorzugsweise als Filtermatte bzw. -membran bzw. - membranschicht(en) ausgebildet. In einer bevorzugten Ausführungsform kann das Filtermedium 16 aus mehreren Schichten bestehen. Üblicherweise ist das Filtermedium 16 im Wesentlichen in vertikaler Richtung VR in dem Prozessiergehäuse 12 angeordnet. In dem Prozessiergehäuse 12 trennt das Filtermedium 16 eine Filtratseite 18 von einer Retentatseite 20. Das Filtermedium 16 ist fluidisch durchlässig, wobei filtermediumsspezifische Stoffe das Filtermedium 16 nicht passieren können. Da der Fluidstrom 14 bestimmungsgemäß von der Retentatseite 20 zur Filtratseite 18 gerichtet ist, verbleiben diese filtermediumsspezifische Stoffe auf der Retentatseite 20 und/oder im Filtermedium 16, gelangen jedoch im Wesentlichen nicht auf die Filtratseite 18 der Prozessiereinheit 10. Zwischen der Retentatseite 20 und der Filtratseite 18 herrscht eine Fluiddruckdifferenz in Abhängigkeit von dem angelegten Fluiddruck und/oder der Durchlässigkeit des Filtermediums 16.

An einem vorzugsweise oberen Ende 22 des Prozessiergehäuses 12 befindet sich zumindest ein Zulaufkanal 24. Dieser erstreckt sich vorzugsweise in im Wesentlichen horizontaler Richtung HR und speist die Prozessiereinheit 10 mit dem zu filtrierenden Medium. Wie in Figur 1 mit Hilfe eines Pfeils gekennzeichnet, strömt der Fluidstrom 14 über den Zulaufkanal 24 in das Prozessiergehäuse 12. In Figur 1 bedeutet das, dass ein Fluidstrom 14 von links in das Prozessiergehäuse 12 strömt. Zumindest ein Teil des Fluidstroms 14 strömt anschließend von der Retentatseite 20 durch das Filtermedium 16 zur Filtratseite 18. Ist die Prozessiereinheit 10 mit einer weiteren Prozessiereinheit (hier nicht gezeigt) parallel verschaltet, strömt ein weiterer Teil des Fluidstroms 14 direkt zu der weiteren Prozessiereinheit ohne das Filtermedium 16 zu durchdringen. Das heißt, dieser Teil des Fluidstroms 14 strömt in den Zulaufkanal 24 der weiteren (nicht gezeigten) Prozessiereinheit. Der Fluidstrom 14, der das Filtermedium 16 durchdrungen hat ("Filtrat"), strömt anschließend in einen Ablaufkanal 26 am vorzugsweise unteren Ende 28 des Prozessiergehäuses 12 und strömt von dort aus dem Prozessiergehäuse 12. Der Ablaufkanal 26 erstreckt sich vorzugsweise ebenfalls im Wesentlichen horizontaler Richtung HR in dem Prozessiergehäuse 12. Das Filtrat, das das Prozessiergehäuse 12 verlässt, kann anschließend in den Ablaufkanal 26 einer weiteren Prozessiereinheit (hier nicht gezeigt) strömen (Parallelschaltung) und/oder zur weiteren Prozessierung in den Zulaufkanal 24 der weiteren Prozessiereinheit (Serienschaltung) strömen.

**Fig. 1b****)** zeigt die Prozessiereinheit 10 aus Fig. 1a), die sich lediglich von dieser unterscheidet, indem das Filtermedium 16 mehrlagig ausgebildet ist.

**Fig. 1c****)** zeigt eine Prozessiereinheit 10, die grundsätzlich ähnlich zu der Prozessiereinheit 10 aus Fig. 1a) ausgebildet ist, sich jedoch in der Art der Filtration unterscheidet. Es werden im Folgenden daher nur die Teile der Prozessiereinheit 10 aus Fig. 1c) beschrieben, die sich von der Prozessiereinheit 10 aus Fig. 1a) unterscheiden.

Im Speziellen ist die Prozessiereinheit aus Fig. 1c) zur Anschwemmfiltration aus gebildet. Dazu ist das Filtermedium 16 als Anschwemmfilter ausgebildet. Das Filtermedium 16 umfasst hier einen Filterträger 17, der vorzugsweise in vertikaler Richtung VR in dem Prozessiergehäuse 12 angeordnet ist und der relativ grob ausgebildet ist. Das Anschwemmmittel wird üblicherweise vor Einbringen in den Filter mit dem Fluid gemischt. Damit wird der Aufbau eines Filterkuchens (hier nicht gezeigt) ermöglicht. Der Filterträger 17 wird so gewählt, dass zumindest ein Filterhilfsmittel zurückgehalten wird. Um in dem Prozessiergehäuse 12 entsprechend Platz für den Filterkuchen bereitzustellen, ist auf der Retentatseite 20 ein Leerraum 19 ausgebildet.

**Fig. 1d****)** zeigt eine weitere Prozessiereinheit 10. Diese ist ähnlich zu der Prozessiereinheit 10 aus Fig. 1a) aufgebaut, weist jedoch anstatt eines Filtermediums 16 ein Schüttgut 21 auf. Im Speziellen kann es sich bei dem Schüttgut 21 um Gele oder Aktivkohle handeln, so dass sich die Prozessiereinheit 10 aus Fig. 1c) zur Chromatographie eignet.

Im Rahmen der Chromatographie können Stoffgemische aufgetrennt werden. Das Schüttgut 21 dient dabei als stationäre Phase, welche unbeweglich in der Prozessiereinheit 10 angeordnet ist. Ein Substanzgemisch wird mit Hilfe einer mobilen Phase (z.B. Wasser) zu der stationären Phase transportiert. Durch eine Wechselwirkung der stationären Phase mit einzelnen Substanzen in der mobilen Phase kann die Durchflusszeit der entsprechenden Substanz durch die Prozessiereinheit 10 verzögert werden, so dass eine Auftrennung von Substanzen ermöglicht wird.

**Fig. 1e** zeigt eine weitere Prozessiereinheit 10. Diese ist ebenfalls ähnlich zu der Prozessiereinheit 10 aus Fig. 1a) aufgebaut, unterscheidet sich jedoch darin, dass ein zweiter Ablaufkanal 27 bereitgestellt ist. Hierdurch eignet sich diese Prozessiereinheit 10 zur Tangentialflussfiltration bzw. Crossflowfiltration. Hierbei wird eine zu filtrierende Suspension mit einer hohen Geschwindigkeit parallel zu dem Filtermedium 16 gepumpt und das Filtrat quer zur Fließrichtung abgezogen. Das Filtrat kann dann über einen der Ablaufkanäle 26 abgeführt werden. Der nicht das Filtermedium 16 durchdringende Anteil des Fluidstroms 14, also das Retentat, kann über den zweiten Ablaufkanal 27 aus der Prozessiereinheit 10 abgeführt werden. Je nach Wunsch kann das Filtrat oder das Rententat aus der Crossflowfiltration im Rahmen des später beschriebenen Prozessiersystems weiter prozessiert werden.

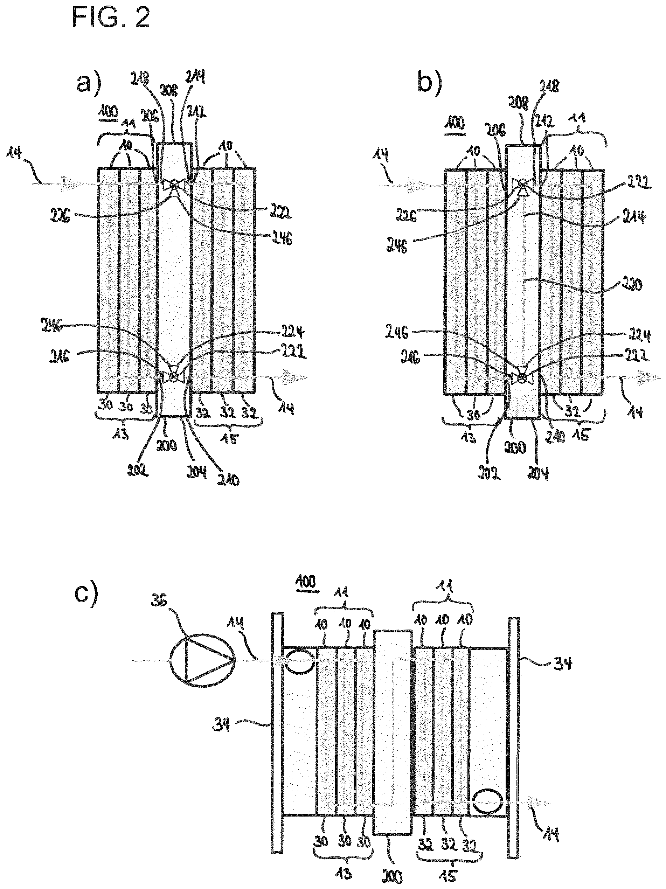

**Figuren 2a)** und **2b****)** zeigen jeweils Prozessiersysteme 100 mit einer Vielzahl von den in den Figuren 1a) bis 1e) beschriebenen Prozessiereinheiten 10. Prozessiereinheiten 10, die direkt miteinander gekoppelt sind, bilden eine Prozessiereinheitengruppe 11. Wie in den Figuren 2a) und 2b) gezeigt, umfassen die Prozessiersysteme 100 jeweils eine erste Prozessiereinheitengruppe 13 und eine zweite Prozessiereinheitengruppe 15, die mit Hilfe einer Adapterplatte 200 gemäß einer Ausführungsform der Erfindung gekoppelt sind. Die in den Figuren 2a) und 2b) direkt gekoppelten Prozessiereinheiten 10 sind als parallel verschaltete Einheiten gezeigt, können jedoch auch seriell verschaltet sein. Die beiden Prozessiereinheitengruppen 11, die mit Hilfe der Adapterplatte 200 gekoppelt sind, können durch die Adapterplatte 200 sowohl parallel (siehe Figur 2a)) als auch seriell (siehe Figur 2b)) verschaltet sein bzw. es kann durch die Adapterplatte 200 zwischen diesen Verschaltungsweisen gewechselt werden. Dies erfolgt in einfacher Weise und ohne konstruktive Änderungen in dem Prozessiersystem 100 durch ein oder mehrere in der Adapterplatte 200 integrierte (bzw. an der Adapterplatte 200 vorgesehene) Umlenkelemente, wie nachfolgend beschrieben. Durch einfaches Umschalten der ein oder mehreren Umlenkelemente, kann zwischen einer parallelen und seriellen Verschaltung der Prozessiereinheitengruppen 11 gewechselt werden. Figur 2a) zeigt eine parallele Verschaltung von Prozessiereinheitengruppen 11, die durch die Adapterplatte 200 gekoppelt sind. Figur 2b) zeigt eine serielle Verschaltung der beiden Prozessiereinheitengruppen 11, die durch die Adapterplatten 200 gekoppelt sind. Obwohl die Figuren 2a) und 2b) Prozessiereinheitengruppen 11 zeigen, die durch die Adapterplatte 200 gekoppelt sind, kann die Adapterplatte 200 auch dazu genutzt werden, nur eine Prozessiereinheit 10 mit einer Prozessiereinheitengruppe 11 oder zwei einzelne Prozessiereinheiten 10 miteinander zu koppeln.

Die Adapterplatte 200 ist vorzugsweise im Wesentlichen plattenförmig ausgebildet und ist von einem Fluidstrom 14 durchströmbar. Vorzugsweise ist die Adapterplatte 200 als Einweg-Bauteil ausgeführt, wobei die Materialeigenschaften vorzugsweise derart gewählt sind, dass eine Sterilisationsmethode wie beispielsweise Gammabestrahlung, Autoklavieren, eine Durchströmung mit Gas wie Ethylenoxid und/oder Heißdampf angewandt werden kann. Insbesondere kann die Adapterplatte aus Kunststoff hergestellt sein. Prozessiereinheiten 10, die in Strömungsrichtung vor einer Adapterplatte 200 angeordnet sind werden als erste Prozessiereinheiten 30 bezeichnet, während Prozessiereinheiten 10, die in Strömungsrichtung nach einer Adapterplatte 200 angeordnet sind als zweite Prozessiereinheiten 32 bezeichnet werden. Mit anderen Worten besteht die erste Prozessiereinheitengruppe 13 aus ersten Prozessiereinheiten 30 und die zweite Prozessiereinheitengruppe 15 aus zweiten Prozessiereinheiten 32, wie in den Figuren 2a) und 2b) gezeigt. Die Adapterplatte 200 ist zwischen der ersten Prozessiereinheitengruppe 13 und der zweiten Prozessiereinheitengruppe 15 angeordnet und verbindet diese fluidisch. Die Adapterplatte 200 umfasst zumindest eine Zutrittsöffnung, über die der Fluidstrom 14 in die Adapterplatte 200 strömen kann. Wie in Figur 2a) gezeigt, weist die Adapterplatte 200 zwei Zutrittsöffnungen auf, nämlich eine erste Zutrittsöffnung 202 am vorzugsweise unteren Ende 204 der Adapterplatte 200, welche mit dem Ablaufkanal 26 der ersten Prozessiereinheit 30 gekoppelt ist, die direkt vor der Adapterplatte 200 angeordnet ist, und eine zweite Zutrittsöffnung 206 am vorzugsweise oberen Ende 208 der Adapterplatte 200, welche mit dem Zulaufkanal 24 dieser ersten Prozessiereinheit 30 gekoppelt ist. Mit anderen Worten kann ein Fluidstrom 14 von der genannten ersten Prozessiereinheit 30 über die erste und zweite Zutrittsöffnung 202 und 206 in die Adapterplatte 200 strömen bzw. fließen. Weiterhin weist die Adapterplatte 200 zumindest eine Austrittsöffnung auf, über die der Fluidstrom 14 aus der Adapterplatte 200 austreten kann. Wie in Figur 2a) gezeigt, weist die Adapterplatte 200 zwei Austrittsöffnungen auf, nämlich eine erste Austrittsöffnung 210, welche vorzugsweise am bzw. nahe einem unteren Ende 204 der Adapterplatte 200 angeordnet ist und mit dem Ablaufkanal 26 der zweiten Prozessiereinheit 32, die direkt nach der Adapterplatte 200 angeordnet ist, gekoppelt ist bzw. werden kann, und eine zweite Austrittsöffnung 212, welche vorzugsweise am bzw. nahe einem oberen Ende 208 der Adapterplatte 200 angeordnet ist und mit dem Zulaufkanal 24 dieser zweiten Prozessiereinheit 32 gekoppelt ist bzw. werden kann. Innerhalb der Adapterplatte 200 erstreckt sich zumindest ein Adapterkanal 214, der die Zutritts- und Austrittsöffnungen miteinander fluidisch verbindet. Im Speziellen weist der Adapterkanal 214 einen ersten Kanalbereich 216 auf, welcher sich zwischen der ersten Zutrittsöffnung 202 und der ersten Austrittsöffnung 210 erstreckt. Ein zweiter Kanalbereich 218 erstreckt sich zwischen der zweiten Zutrittsöffnung 206 und der zweiten Austrittsöffnung 212. Der erste und zweite Kanalbereich 216 und 218 wird über einen Verbindungskanalbereich 220 fluidisch verbunden.

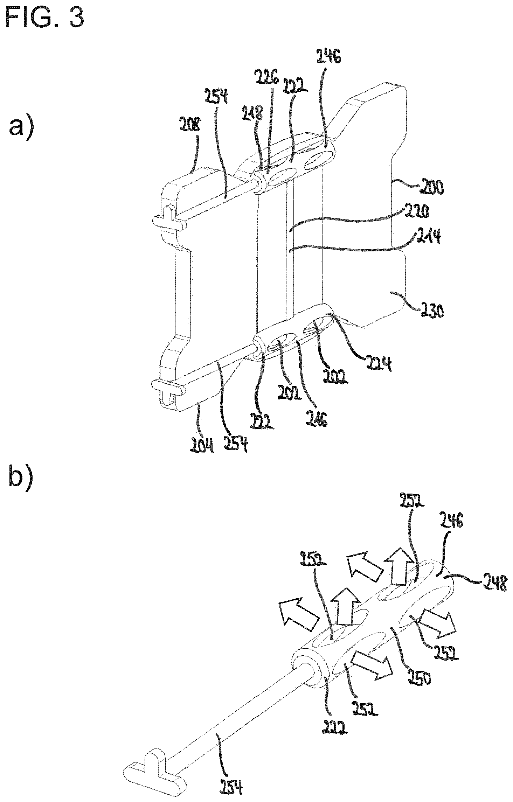

Wie in den Figuren 2a) und 2b) gezeigt, sind in dem Adapterkanal 214 zumindest zwei Umlenkelemente angeordnet. Im Speziellen handelt es sich hierbei um Ventile 222. In einer bevorzugten Ausführungsform ist ein erstes Ventil 224 in dem ersten Kanalbereich 216 und ein zweites Ventil 226 in dem zweiten Kanalbereich 218 angeordnet. In einer konkreten Ausführungsform können die Ventile 222 als Mehrwegventile 246 ausgebildet sein, wie dies im Folgenden anhand der Figuren 3a) und 3b) beschrieben wird.

Die zumindest eine erste und zweite Prozessiereinheit 30 und 32 können baugleich ausgebildet sein oder verschieden sein, um unterschiedliche Prozessarten durchführen zu können. Vorzugsweise sind die ersten Prozessiereinheiten 30 in der ersten Prozessiereinheitengruppe 13 und die zweiten Prozessiereinheiten 32 der zweiten Prozessiereinheitengruppe 15 baugleich ausgebildet. Es ist jedoch auch denkbar, dass sich die ersten Prozessiereinheiten 30 sowie die zweiten Prozessiereinheiten 32 sich jeweils untereinander unterscheiden. Beispielsweise kann die zumindest eine erste Prozessiereinheit 30 als Tiefenfilter ausgebildet sein, während die zumindest eine zweite Prozessiereinheit 32 als Sterilfilter ausgebildet sein kann.

**Fig. 2c****)** zeigt das Prozessiersystem 100 aus Fig. 2b), in dem die einzelnen Prozessiereinheiten 10 und die Adapterplatte 200 mit Hilfe von Abschlusshalterungen 34 zusammengehalten werden. Mit anderen Worten befindet am Eintrittspunkt, an dem der Fluidstrom 14 in das Prozessiersystem 100 eintritt, und am Austrittspunkt, an dem der Fluidstrom 14 aus dem Prozessiersystem 100 austritt, jeweils eine Abschlusshalterung 34. Diese kann vorzugsweise als Abschlussplatte ausgebildet sein, so dass die einzelnen Prozessiereinheiten 10 sandwichartig zwischen diesen Abschlussplatten gehalten werden. Ein- bzw. Auslässe können Teil der Abschlusshalterung 34 sein. Insbesondere kann aber zwischen einer Abschlusshalterung 34 und der jeweils angrenzenden Prozessiereinheit 10 eine weitere Abschlussplatte mit entsprechenden Anschlüssen eingebracht werden. Vorzugsweise wird der Fluidstrom 14 mit Hilfe einer Zuflusspumpe 36 in das Prozessiersystem 100 gepumpt.

**Figur 3a****)** zeigt eine Schnittansicht durch eine Adapterplatte 200. Wie in Figur 3a) gezeigt, ist die Adapterplatte 200 vorzugsweise zweiteilig ausgebildet und umfasst eine Zulaufplatte 230 und eine Ablaufplatte (hier nicht gezeigt). Als Zulaufplatte 230 wird die Platte der Adapterplatte 200 bezeichnet, in der die zumindest eine Zutrittsöffnung angeordnet ist. Als Ablaufplatte wird die Platte der Adapterplatte 200 bezeichnet, in der die zumindest eine Austrittsöffnung angeordnet ist. Die Form und/oder Größe der Zulaufplatte 230 und Ablaufplatte können identisch sein. In dem ersten und/oder zweiten Kanalbereich 216 und 218 befindet sich jeweils ein Mehrwegventil 246, das als Umlenkelement dient. Das Mehrwegventil 246 umfasst ein Ventilrohr 248, das sich jeweils zumindest teilweise in dem ersten und zweiten Kanalbereich 216 und 218 erstreckt und dort verlagerbar (insbesondere rotierbar bzw. drehbar) gelagert ist. Eine Verlagerung (insbesondere Rotation) kann per Hand mittels eines Handstückes 254 erfolgen, das aus der Adapterplatte 200 ragt (siehe Figuren 3a) und 3b)) und/oder kann durch ein externes Steuergerät (nicht gezeigt) erfolgen.

In einer Mantelfläche 250 des Ventilrohrs 248 sind zumindest zwei Ventilöffnungen 252 ausgebildet. Diese sind in Bezug auf die Verlagerungsrichtung (insbesondere Rotationsrichtung) derart voneinander versetzt angeordnet, dass in einer ersten Stellung des Ventilrohrs 248 ein erster Fluidstrom bzw. ein zweiter Fluidstrom ermöglicht wird. Das heißt, dass beispielsweise der erste Fluidstrom über die erste Zutrittsöffnung 202 der Adapterplatte 200 durch eine erste Ventilöffnung 252 die zumindest teilweise mit der ersten Zutrittsöffnung 202 überlappt, in das Ventilrohr 248 eintritt.

Die zweite Ventilöffnung 252 überlappt zumindest teilweise die erste Austrittsöffnung 210, so dass der erste Fluidstrom über die erste Austrittsöffnung 210 aus der Adapterplatte 200 strömen kann. Dies gilt in entsprechender Weise für den zweiten Fluidstrom und der zweiten Zutritts- und Austrittsöffnung 206 und 212. Ein Fluidstrom 14 durch den Verbindungskanalbereich 220 wird aufgrund der Stellung der Ventilöffnungen 252 in den jeweiligen Kanalbereichen blockiert. Da der erste und zweite Fluidstrom durch die Mehrwegventile 246 jedoch gestattet wird, sind die erste und zweite Prozessiereinheit 30 und 32, die jeweils direkt mit der Adapterplatte 200 gekoppelt sind, parallel miteinander verschaltet.

Vorzugsweise weisen die Ventilrohre 248 jedoch zumindest drei Ventilöffnungen 252 auf, wie in den Figuren 3a) und 3b) gezeigt. Diese sind derart angeordnet, dass in einer ersten Ausrichtung (insbesondere Rotationsstellung) des Ventilrohrs 248 zumindest zwei Ventilöffnungen 252 derart ausgerichtet sind, dass die oben beschriebenen ersten und zweiten Fluidströme gestattet werden und so die erste und zweite Prozessiereinheit 30 und 32, die jeweils direkt mit der Adapterplatte gekoppelt sind, parallel verschaltet sind. Ein Fluidstrom 14 durch den Verbindungskanalbereich 220 wird jedoch unterbunden, da keine Ventilöffnung 252 zumindest teilweise mit dem Verbindungskanalbereich 220 überlappt.

Aufgrund der Anordnung der zumindest drei Ventilöffnungen 252 sind in einer zweiten Ausrichtung (insbesondere Rotationsstellung) des jeweiligen Ventilrohrs 248 jedoch entweder der Durchgang zu einer Zutrittsöffnung oder zu einer Austrittsöffnung blockiert, während der Zugang zu dem Verbindungskanalbereich 220 gestattet wird. Dies ermöglicht eine serielle Verschaltung der ersten und zweiten Prozessiereinheit 30 und 32.

Mit anderen Worten sind in einer seriellen Verschaltung die Mehrwegventile 246 derart auszurichten, dass ein Fluidstrom 14 über die erste Zutrittsöffnung 202 der Adapterplatte 200 und über eine Ventilöffnung 252, die zumindest teilweise mit der ersten Zutrittsöffnung 202 überlappt, in das erste Ventilrohr 248 eintreten kann. Über eine Ventilöffnung 252, die zumindest teilweise mit dem Verbindungskanalbereich 220 überlappt, kann der Fluidstrom 14 in den Verbindungskanalbereich 220 strömen. Der Durchgang zu der ersten Austrittsöffnung 210 wird durch das Ventilrohr 248 blockiert, da keine Ventilöffnung 252 mit der ersten Austrittsöffnung 210 überlappt. Anschließend kann der Fluidstrom 14 über eine Ventilöffnung 252 des zweiten Ventilrohrs 248, die zumindest teilweise mit dem Verbindungskanalbereich 220 überlappt, in das zweite Ventilrohr 248 einströmen. Über eine Ventilöffnung 252 in dem zweiten Ventilrohr 248, die zumindest teilweise mit der zweiten Austrittsöffnung 212 der Adapterplatte 200 überlappt, kann der Fluidstrom 14 aus der Adapterplatte 200 ausströmen. Der Durchgang zu der zweiten Zutrittsöffnung 206 wird durch das Ventilrohr 248 blockiert, da keine Ventilöffnung 252 mit der zweiten Zutrittsöffnung 206 überlappt.

Somit kann in einfacher Weise und ohne konstruktive Umbaumaßnahmen zwischen einer seriellen und parallelen Verschaltung zweier Prozessiereinheiten 10 oder Prozessiereinheitengruppen 11 gewechselt werden. Insbesondere ist die Adapterplatte 200 als kompaktes Bauelement ausgelegt, so dass das Prozessiersystem 100 einen geringen Platzbedarf aufweist. Die Ventile 222 können als kostengünstige Einweg-Bauteile ausgelegt sein. Mögliche Anwendungen können sein: Umschaltung für Tangentialstromfiltration von Standardbetrieb zu SinglePass, Umschaltung zwischen parallel und seriell verschalteten Chromatographieeinheiten beispielsweise zur seriellen Kombination von unterschiedlichen Chromatographiemedien oder zur Verbesserung der Kapazitätsausnutzung.

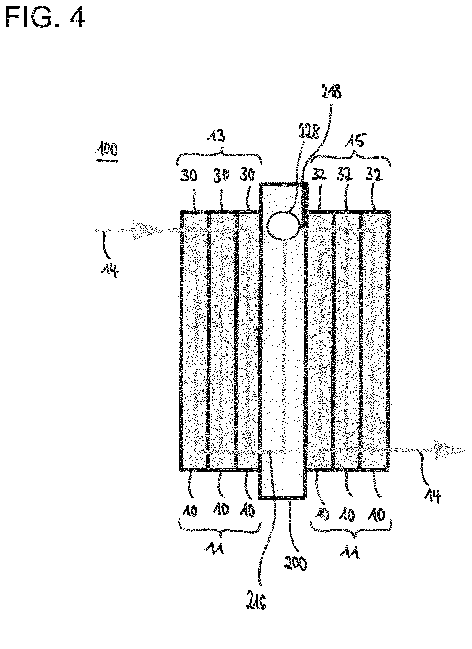

**Figur 4** zeigt eine Ausführungsform eines Prozessiersystems 100, bei dem in bzw. an der Adapterplatte 200 zumindest ein Sensor 228 angeordnet ist. Dieser Sensor 228 ist dabei derart ausgebildet, dass beispielsweise der Druck, der Volumenstrom, der UV-Wert, der pH-Wert, die Trübung und/oder die Viskosität des Fluidstroms 14 gemessen werden kann. Die gemessenen Werte können dazu verwendet werden, den Filtrationsvorgang wiederum zu steuern bzw. zu regeln. Hierzu können die gemessenen Werte an ein externes Steuergerät (nicht gezeigt) übermittelt werden. Der Sensor 228 kann als kostengünstiges Einweg-Bauteil ausgebildet sein.

Die gezeigte Adapterplatte 200 koppelt die erste und zweite Prozessiereinheitengruppe 13 und 15 hier seriell. Es ist jedoch ebenfalls möglich, Sensoren 228 bei einer parallelen Verschaltung zweier Prozessiereinheitengruppen 11 zu verwenden. Hierbei ist es jedoch notwendig, dass der oder die Sensor(en) 228 in oder an dem ersten und/oder zweiten Kanalbereich 216 und 218 angeordnet ist/sind, um einen Kontakt zum Fluidstrom 14 herstellen zu können. Sind die ersten und zweiten Prozessiereinheitengruppen 13 und 15 seriell verschaltet, kann der Sensor 228 auch in bzw. an dem Verbindungskanalbereich 220 angeordnet sein.

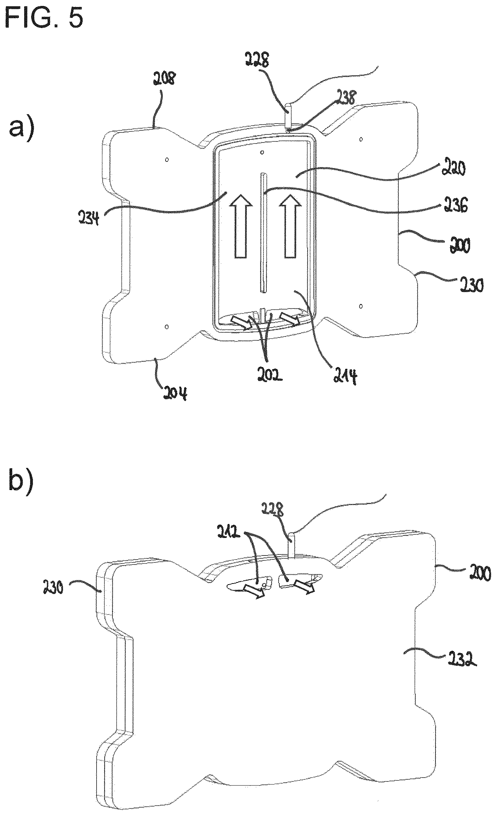

**Figuren 5a) und 5b****)** zeigen eine bevorzugte Ausführungsform einer Adapterplatte 200, in der zumindest ein Sensor 228 integriert ist. Wie in Figur 5b) gezeigt, ist die Adapterplatte 200 ebenfalls vorzugsweise zweiteilig bzw. mehrteilig ausgebildet. Die Zulaufplatte 230 umfasst die zumindest eine erste Zutrittsöffnung 202, vorzugsweise am unteren Ende 204 der Adapterplatte 200. Wie in Figur 5a) gezeigt, umfasst die Zulaufplatte 230 zwei erste Zutrittsöffnungen 202. Sie weist jedoch keine zweite(n) Zutrittsöffnung(en) 206 auf. Da die gezeigte Adapterplatte 200 ausschließlich eine serielle Verschaltung zweier Prozessiereinheiten 10 ermöglichen soll, sind diese jedoch auch nicht zwingend erforderlich. Für eine parallele Verschaltung könnte zumindest eine zweite Zutrittsöffnung 206, vorzugsweise am oberen Ende 208 der Adapterplatte 200, in der Zulaufplatte 230 ausgebildet sein.

Wie ferner in Figur 5b) gezeigt, umfasst die Ablaufplatte 232 zumindest eine zweite Austrittsöffnung 212. Im konkreten Fall der Figur 5b) weist die Ablaufplatte 232 zwei zweite Austrittsöffnungen 212 auf. Sollte eine parallele Verschaltung zweier Prozessiereinheiten 10 gewünscht sein, kann die Ablaufplatte 232 zusätzlich zumindest eine erste Austrittsöffnung 210 aufweisen. Die Zu- und Ablaufplatte 230 und 232 können miteinander verschraubt und/oder verklebt und/oder verschweißt und/oder verklickt sein.

In der Zu- und/oder Ablaufplatte 230 und 232 kann eine entsprechende Kanalvertiefung 234 ausgebildet sein. Sind die Zu- und Ablaufplatte 230 und 232 zusammengesetzt, ist so ein Adapterkanal 214 ausgebildet, der es ermöglicht, dass die Adapterplatte 200 von einem Fluidstrom 14 durchströmbar ist. Vorzugsweise ist in dem Verbindungskanalbereich 220 des Adapterkanals 214 zumindest ein Strömungssteg 236 ausgebildet, der sich vorzugsweise im Wesentlichen in Strömungsrichtung des Fluidstroms 14 erstreckt und dazu beiträgt, den Fluidstrom 14 in verbesserter Weise zu lenken. Der Sensor 228 kann in die Zu- und/oder Ablaufplatte 230 und 232 integriert sein und in den Adapterkanal 214 ragen, so dass ein Messelement des Sensors 228 mit dem Fluidstrom 14 in Kontakt treten kann. Vorzugsweise umfasst der Adapterkanal 214 zumindest bereichsweise einen Verbindungskanal 238 zum Sensor 228. Dieser Verbindungskanal 238 kann eine zumindest bereichsweise Vertiefung in dem Adapterkanal 214 sein, in die der Sensor 228 zumindest teilweise hineinragt.

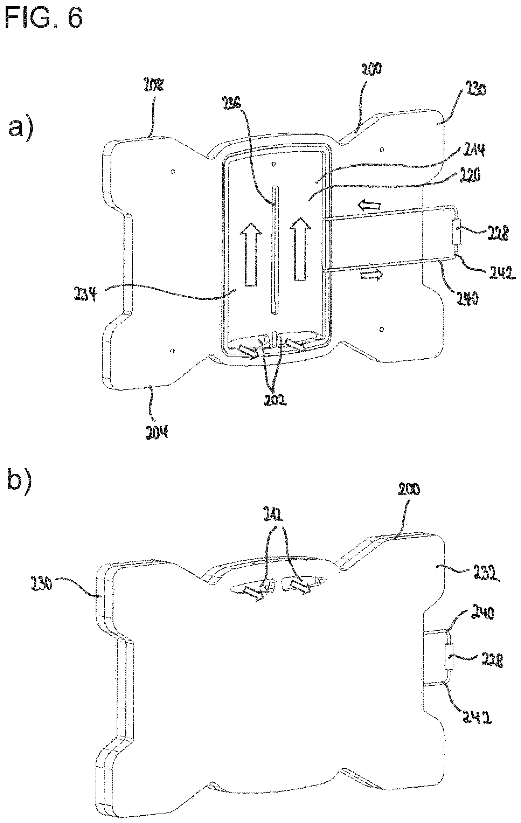

**Figuren 6a) und 6b****)** zeigen die Adapterplatte 200 aus den Figuren 5a) und 5b), jedoch ist in dieser Ausführungsform der Sensor 228 nicht in oder an dem Adapterkanal 214 angeordnet, sondern in bzw. an einer Hilfsabzweigung 240. Bei der Hilfsabzweigung 240 handelt es sich um eine Abzweigung aus dem Adapterkanal 214, in den zumindest ein Teil des Fluidstroms 14 abgezweigt wird. Dieser Teil des Fluidstroms 14 strömt zumindest für eine bestimmte Zeit aus dem Adapterkanal 214, um vorzugsweise später wieder in den Adapterkanal 214 zurückgeführt zu werden.

Die Hilfsabzweigung 240 kann dazu genutzt werden, kleine Mengen des Fluidstroms 14 für entsprechende Messungen abzuzweigen. Die Hilfsabzweigung 240 kann mit Hilfe eines separaten Kanalelements 242 ausgebildet sein, das zumindest teilweise in die Adapterplatte 200 integriert ist. Wie in den Figuren 6a) und 6b) gezeigt, kann ein Teil der Hilfsabzweigung 240 aus der Adapterplatte 200 herausragen. Insbesondere kann hierdurch auch der Sensor 228 außerhalb der Adapterplatte 200 angeordnet sein. Diese Anordnung wird jedoch auch durch die Beschreibung "an dem Adapterkanal 214 angeordnet" umfasst.

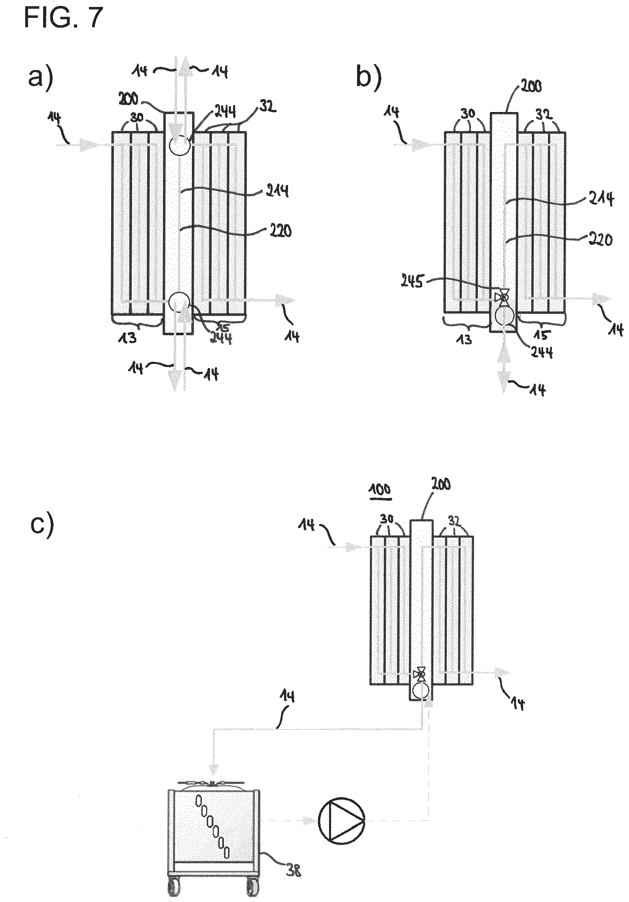

**Figur 7a****)** zeigt ein Prozessiersystem 100 mit einer ersten und zweiten Prozessiereinheitengruppe 13 und 15, die mittels einer Adapterplatte 200 gekoppelt sind. Die verknüpften Prozessiereinheitengruppen 11 sind in der gezeigten Ausführungsform seriell durch die Adapterplatte 200 verschaltet, es ist jedoch ebenfalls möglich, die beiden Prozessiereinheitengruppen 11 parallel gemäß der übrigen beschriebenen Ausführungsformen zu verschalten.

In der Adapterplatte 200 kann gemäß Figur 7a) zumindest ein Hilfsausgang- und/oder -zugang 244 ausgebildet sein. Über einen Hilfsausgang kann zumindest ein Teil des Fluidstroms 14 aus dem Adapterkanal 214 entnommen werden bzw. herausströmen. Über einen Hilfszugang kann der entnommene Fluidstrom 14 wieder zurück in den Adapterkanal 214 strömen oder Zugriff zu dem Adapterkanal 214 gewährleistet werden. Die Verwendung mindestens eines Hilfsausgangs bzw. -zugangs ermöglicht beispielsweise die externe Zuschaltung einer Pumpe, einen Integritätstest an unterschiedlichen Prozessiereinheiten 10 des Prozessiersystems 100, die Abfuhr des Fluids aus der ersten oder weiteren vorgeschalteten Prozessiereinheiten 30 zur zwischenzeitlichen Pufferung oder zur weiteren Prozessierung, eine Entlüftung und/oder eine Probeentnahme. Insbesondere kann zumindest ein Diafiltrationsmedium und/oder andere Reagenzien über einen Hilfszugang zugegeben werden. Figur 7a) zeigt eine Adapterplatte 200 mit zwei Hilfsaus- und -zugängen 244. Mögliche Anschlüsse an einem Hilfsaus- und Hilfszugang 244 könnten Triclamps oder Sterilkonnektoren sein.

**Figur 7b****)** zeigt das Prozessiersystem 100 aus Fig. 7a), jedoch mit nur einem Hilfsaus- bzw. zugang 244. Die erste und zweite Prozessiereinheitengruppe 13 und 15 sind hier ebenfalls seriell verschaltet, insbesondere handelt es sich hier um eine serielle Verschaltung von Chromatographieschritten. Die zumindest eine erste Prozessiereinheit 30 ist hier vorzugsweise eine Bind&Elute Einheit. Ein Chromatographieschritt kann beispielsweise eine Affinitätschromatographie mit Protein A Ligand gekoppelt auf Membranen (Membranadsorber) oder Gelen sein. Um die zumindest eine zweite Prozessiereinheit 32 nicht zu kontaminieren, kann zumindest ein Hilfsausgang 244 in der Adapterplatte 200 vorgesehen sein, so dass ein Fluidstrom 14 aus dem Ablaufkanal 26 der ersten Prozessiereinheit 30, die direkt mit der Adapterplatte 200 gekoppelt ist, über den Hilfsausgang 244 abströmen kann. Insbesondere können über den Hilfsausgang 244 diverse Puffer und Waschlösungen abgeführt werden.

Vorzugsweise ist an dem Hilfsausgang 244 ein Hilfsventil 245 angeordnet, um den Fluidstrom 14 zwischen dem Hilfsausgang 244 und der zweiten Prozessiereinheit 32, die direkt mit der Adapterplatte 200 gekoppelt ist, umzulenken.

Eine mögliche Abfolge des Prozesses in dem Prozessiersystem 100 der Figur 7b) könnte sein:
(1) Equilibrierung: Eine Pufferlösung wird verwendet, um das Chromatographiemedium zu reinigen und in den gewünschten Gleichgewichtszustand zu bringen; die austretende Pufferlösung aus der ersten Prozessiereinheit 30, die direkt mit der Adapterplatte 200 gekoppelt ist, wird über den Hilfsausgang 244 der Adapterplatte 200 abgeleitet.
(2) Beladen mit dem zu verarbeitenden Fluidstrom 14: Das Zielmolekül aus dem Fluidstrom 14 wird im Chromatographiematerial der ersten Prozessiereinheitengruppe 13 gebunden, die restliche Flüssigkeit mit Verunreinigungen tritt aus der ersten Prozessiereinheit 30, die direkt mit der Adapterplatte 200 gekoppelt ist, über den Hilfsausgang 244 der Adapterplatte 200 aus;
(3) Waschen: Pufferlösung wird verwendet, um verbleibende Verunreinigungen aus der ersten Prozessiereinheitengruppe 13 auszuspülen; die austretende Pufferlösung mit den Verunreinigungen wird über den Hilfsausgang 244 der Adapterplatte 200 abgeleitet.
(4) Elution: Mit einer weiteren Pufferlösung wird das Zielmolekül vom Chromatographiematerial der ersten Prozessiereinheitengruppe 13 wieder gelöst und kann direkt in die zweite Prozessiereinheitengruppe 15 über die zweite Prozessiereinheit 32, die direkt mit der Adapterplatte 200 gekoppelt ist, geleitet werden (der Hilfsausgang 244 ist dabei geschlossen); die zweiten Prozessiereinheiten 32 sind dabei Chromatographie-Einheiten, welche je nach Prozessführung weitere Verunreinigungen (Flow-Through Polishing) oder das Zielmolekül binden (Bind&Elute) können.
(5) Reinigen/Regenerieren/Dekontaminieren: Mit einer weiteren Lösung wird das Chromatographiematerial der ersten Prozessiereinheitengruppe 13 behandelt; die austretende Lösung mit den Verunreinigungen wird über den Hilfsausgang 244 der Adapterplatte 200 abgeleitet.

Der Hilfszu- und -abgang 244 kann des Weiteren verwendet werden, um mindestens einen der in der vorherigen Abfolge beschriebenen Schritte getrennt von der ersten Prozessiereinheitengruppe 13 durchzuführen. Die Adapterplatte 200 wird dabei genutzt, um entsprechende Lösungen einzuspeisen, um mindestens eine zweite Prozessiereinheit 32 zu behandeln.

Die beschriebene Abfolge von Schritten kann beliebig erweitert oder reduziert oder kombiniert werden.

In Schritt (4) kann das Zielmolekül für eine Zwischenlagerung und/oder für weitere Prozessieren (z.B. Virusinaktivierung bei niedrigen pH-Werten in einem Mischtank 38) auch über den Hilfsausgang 244 abgeleitet werden, wie in **Figur 7c****)** gezeigt. Später wird dieses dann wieder der zweiten Prozessiereinheitengruppe 15 zugeführt.

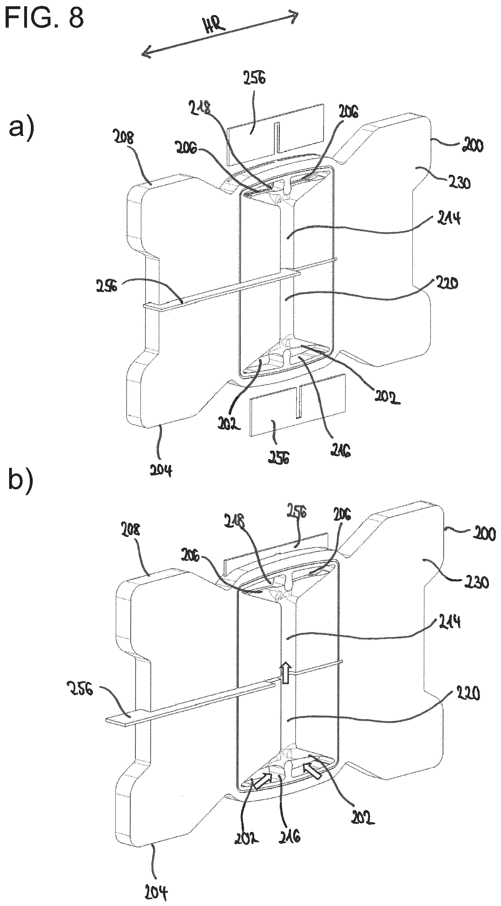

**Figuren 8a) und 8b****)** zeigen eine weitere Ausführungsform einer zweiteiligen Adapterplatte 200 mit zumindest einem Umlenkelement. Im Speziellen ist das Umlenkelement gemäß dieser Ausführungsform verschiebbar in der Adapterplatte 200 gelagert. Insbesondere ist das Umlenkelement (im Folgenden als "Verschlusselement 256" bezeichnet) derart verschiebbar bzw. verlagerbar in der Adapterplatte 200 gelagert, dass in einer ersten Position des Verschlusselements 256, das Verschlusselement 256 zumindest teilweise in den Adapterkanal 214 hineinragt, um einen Fluidstrom 14 durch den Adapterkanal 214 vollständig oder zumindest teilweise zu blockieren (siehe Figur 8a)). In einer zweiten Position des Verschlusselements 256 ragt das Verschlusselement 256 nicht in den Adapterkanal 214, so dass ein Fluidstrom 14 im Wesentlichen ungehindert strömen kann.

Das Verschlusselement 256 ist vorzugsweise als Platte ausgebildet. Ein entsprechend ausgebildeter Schlitz bzw. Ausnehmung ist für das entsprechende Verschlusselement 256 in der Adapterplatte 200 ausgebildet und das Verschlusselement 256 ist in diesem Schlitz verlagerbar bzw. verschiebbar gelagert. Es kann ein Verschlusselement 256 vorgesehen sein, das derart angeordnet ist, dass ein Fluidstrom 14 durch den Verbindungskanalbereich 220 blockierbar ist. Hierzu ist das Verschlusselement 256 in den Verbindungskanalbereich 220 vorzugsweise in horizontaler Richtung HR einschiebbar bzw. eingesetzt.

Weiterhin können je ein Verschlusselement 256 vorgesehen sein, um in den ersten und/oder zweiten Kanalbereich 216 und 218 einschiebbar bzw. einsetzbar zu sein. Das heißt, dass ein Verschlusselement 256 von oben bzw. von unten in den jeweiligen Kanalbereich einschiebbar bzw. einsetzbar ist. Insbesondere ist ein Verschlusselement 256 derart in den ersten Kanalbereich 216 einschiebbar, dass die zumindest eine erste Austrittsöffnung 210 durch das Verschlusselement 256 blockierbar ist. Das weitere Verschlusselement 256 ist derart in den zweiten Kanalbereich 218 einschiebbar, dass die zumindest eine zweite Zutrittsöffnung 206 durch das Verschlusselement 256 blockierbar ist. Mit anderen Worten kann durch die genannten Verschlusselementen 256 die erste und zweite Prozessiereinheitengruppe 13 und 15 bzw. eine erste und zweite Prozessiereinheit 30 und 32 parallel oder seriell verschaltet werden.

Figur 8a) zeigt eine parallele Verschaltung der Prozessiereinheiten 10, indem ein Verschlusselement 256 in den Verbindungskanalbereich 220 eingeschoben bzw. eingesetzt ist und ein Fluidstrom 14 durch den Verbindungskanalbereich 220 blockiert ist. In Figur 8b) wird eine serielle Verschaltung der Prozessiereinheiten 10 gezeigt, indem Verschlusselemente 256 in den ersten und zweiten Kanalbereich 216 und 218 eingeschoben sind. Der Fluidstrom 14 durch den Verbindungskanalbereich 220 ist jedoch nicht durch ein Verschlusselement 256 blockiert.

Die Verschlusselemente 256 sind händisch verschiebbar bzw. verlagerbar und/oder können über eine externe Steuerung (nicht gezeigt) und einem gekoppelten Motor (nicht gezeigt) verschoben werden.

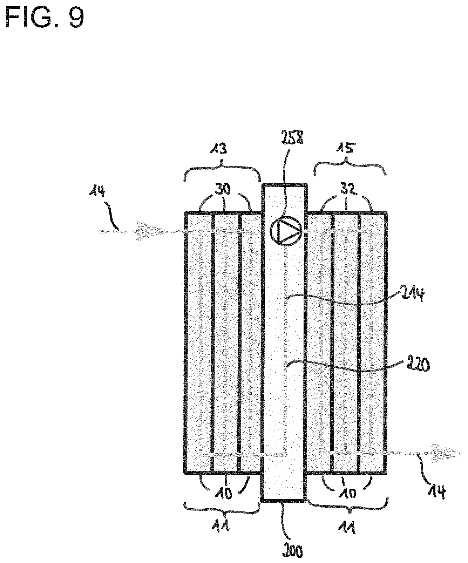

**Figur 9** zeigt ein Prozessiersystem 100 mit einer ersten und zweiten Prozessiereinheitengruppe 13 und 15. Die erste und zweite Prozessiereinheitengruppe 13 und 15 sind mittels einer Adapterplatte 200 gekoppelt. In bzw. an dem Adapterkanal 214 der Adapterplatte 200 ist zumindest eine Pumpe 258 angeordnet. Diese kann bei einer parallelen Verschaltung zweier Prozessiereinheitengruppen 11 genutzt werden, ist jedoch besonders in einer seriellen Verschaltung, wie in Figur 9 gezeigt, vorteilhaft, da hierdurch beispielsweise ein Flusswiderstand überwunden werden kann und somit eine hinreichende Filtrationsleistung in der zweiten Prozessiereinheitengruppe 15 erzielt werden kann.

Weiterhin kann die Pumpe 258 insbesondere als Verdrängerpumpe ausgebildet sein, um in der ersten Prozessiereinheitengruppe 13 einen Unterdruck zu initiieren und in der zweiten Prozessiereinheitengruppe 15 einen gewünschten Filtrationsdruck aufzubauen. Insbesondere kann die Pumpe 258 als kostengünstiges Einweg-Bauteil ausgebildet sein.

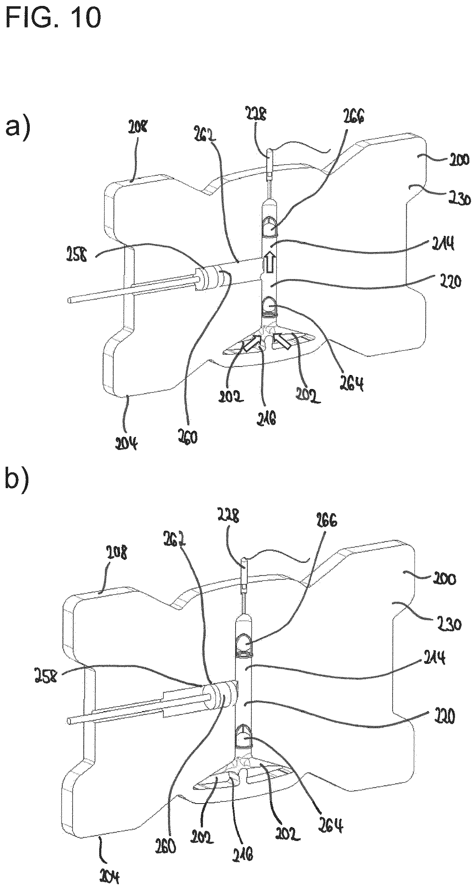

**Figuren 10a) und 10b****)** zeigen eine konkrete Ausführungsform einer Adapterplatte 200 mit einer Verdrängerpumpe, nämlich einer Kolbenpumpe. Die Pumpe 258 ist vorzugsweise in dem Verbindungskanalbereich 220 angeordnet und umfasst zumindest einen Kolben 260, welcher ausgelegt ist, eine Hub-Bewegung, also eine geradlinige (translatorische) Bewegung, auszuführen. Hierzu ist der Kolben 260 in einem Zylinder 262 gelagert. Zusätzlich weist die Pumpe 258 einen Zu- und einen Ablauf auf, die jeweils durch ein Ventil verschließbar sind.

Im Speziellen ist ein Abschnitt in dem Verbindungskanalbereich 220 in Flussrichtung durch ein Einlassventil 264 und durch ein Auslassventil 266 abgetrennt bzw. abgegrenzt. Über das Einlassventil 264 kann das Fluid in diesen Abschnitt strömen und über das Auslassventil 266 entweichen. Der Zylinder 262, in dem der Kolben 260 gelagert ist und in diesem eine translatorische Bewegung ausführen kann, ist derart angeordnet, dass der Zylinder 262 an dem Verbindungskanalbereich 220 angeordnet und mit diesem fluidisch verbunden ist. Insbesondere ist der Zylinder 262 zwischen dem Einlass- und Auslassventil 264 und 266 an dem Verbindungskanalbereich 220 angeordnet.

In einem ersten Takt, beim Ansaugen, führt der Kolben 260 eine Rückwärtsbewegung aus, also eine Bewegung weg von dem Verbindungskanalbereich 220. Das Einlassventil 264 öffnet sich und das zu fördernde Fluid kann in den Verbindungskanalbereich 220 bzw. den Zylinder 262 strömen. In einem zweiten Takt, bei der Förderbewegung schließt sich das Einlassventil 264 und der Kolben 260 bewegt sich in Richtung zu dem Verbindungskanalbereich 220. Es öffnet sich das Auslassventil 266 und das Fördermedium wird herausgedrückt.

Vorzugsweise ist zumindest ein Sensor 228 in dem Adapterkanal 214 angeordnet, um den Fluidstrom 14 zu überwachen. Hierzu kann der Sensor 228 vorzugsweise in Flussrichtung nach der Pumpe 258 angeordnet sein, um beispielsweise den Fluiddruck oder den Fluidfluss zu messen. Dieser Wert kann dazu verwendet werden, mittels eines externen Steuergeräts die Pumpe 258 zu regeln.

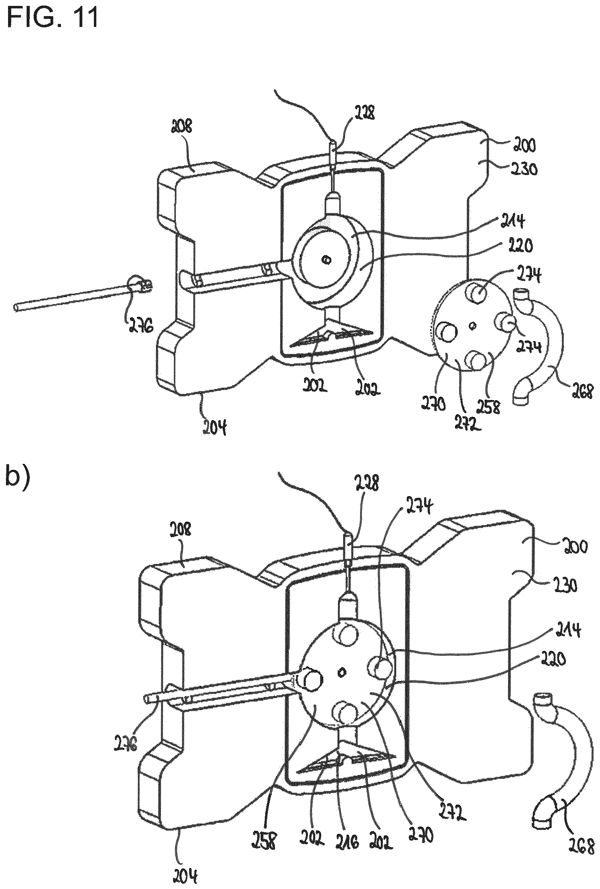

Eine weitere Möglichkeit einer Verdrängerpumpe wird anhand der **Figuren 11a) und** **11b****)** gezeigt. Die in die Adapterplatte 200 integrierte Pumpe 258 ist eine Peristaltikpumpe. Diese ist vorzugsweise aus den bereits oben beschriebenen Gründen in dem Verbindungskanalbereich 220 angeordnet.

Eine Peristaltikpumpe, auch Schlauchpumpe genannt, ist eine Verdrängerpumpe, bei der das zu fördernde Fluid durch äußere mechanische Verformung eines Schlauches 268 durch diesen hindurchgedrückt wird. Der Schlauch 268 stellt in dem vorliegenden Fall somit einen Teil des Verbindungskanalbereichs 220 dar, durch den das Fluid in der Adapterplatte 200 strömt. Der Bereich, in dem der Schlauch 268 angeordnet ist, ist kreisförmig bzw. der Verbindungskanalbereich 220 weitet sich bogenförmig auf. In dem kreisförmigen Abschnitt des Verbindungskanalbereichs 220 ist ein Rotor 270 angeordnet, der dort drehbar gelagert ist.

Der Rotor 270 ist vorzugsweise als kreisrunde Platte ausgebildet. Auf einer Oberseite 272 des Rotors 270 ist/sind zumindest eine Rolle 274 und/oder ein Gleitschuh angeordnet. Der Schlauch 268 liegt zumindest bereichsweise an einer Mantelfläche des kreisförmigen Abschnitts des Verbindungskanalbereichs 220 an. Durch eine Rotation des Rotors 270 ist der Schlauch 268 von innen durch die Rollen 274 und/oder Gleitschuhe abklemmbar. Dies führt dazu, dass sich eine Abklemmstelle entlang des Schlauches 268 bewegt und dadurch das zu fördernde Fluid vorantreibt. Der Rotor 270 ist über einen Zahnradmechanismus 276, wie in den Figuren 11a) und 11b) gezeigt, drehbar. Zumindest ein Sensor 228 kann ähnlich zu der Ausführungsform der Figuren 10a) und 10b) in der Adapterplatte 200 angeordnet sein.

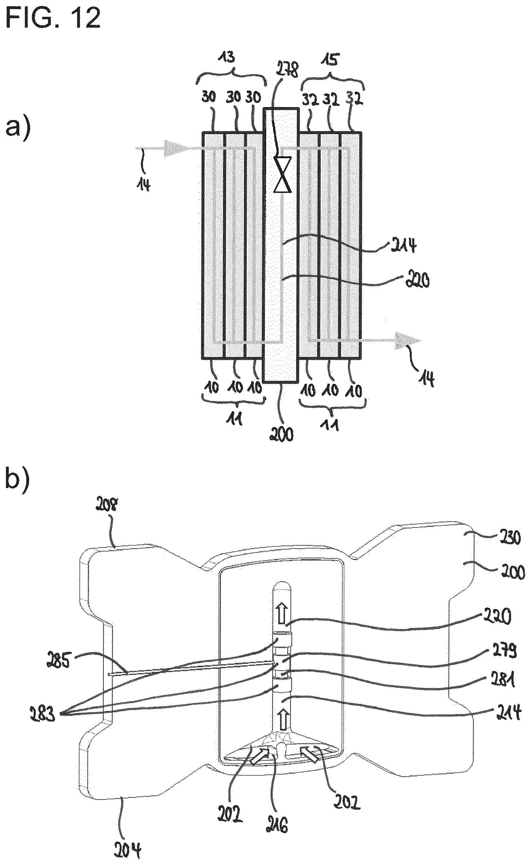

**Figur 12a****)** zeigt ein Prozessiersystem 100 mit einer ersten und zweiten Prozessiereinheitengruppe 13 und 15, die durch eine Adapterplatte 200 gekoppelt sind. In der Adapterplatte 200 dieser Ausführungsform ist ein Flussbegrenzer 278 integriert. Dieser kann in vorteilhafter Weise dazu verwendet werden, den Fluss und/oder Druck des Fluidstroms 14 zu regulieren. Dies ist insbesondere bei einer seriellen Verschaltung zweier Prozessiereinheitengruppen 11 bzw. Prozessiereinheiten 10 sinnvoll, wie in Figur 12a) gezeigt.

**Figur 12b****)** zeigt die Adapterplatte 200 aus Figur 12a) in einer Schnittansicht mit einem Quetschventil als Flussbegrenzer 278. In dem Verbindungskanalbereich 220 ist hierzu vorzugsweise zumindest ein flexibles Sperrelement 279 zumindest bereichsweise angeordnet. Das flexible Sperrelement 279 ist kanalförmig ausgebildet, so dass in einer ersten Position der Fluidstrom 14 ungehindert durch das flexible Sperrelement 279 hindurchströmen kann. Vorzugsweise ist das Sperrelement 279 als Schlauchelement, insbesondere als Silikonschlauch ausgebildet. Wie in Figur 12b) gezeigt, kann das Sperrelement 279 an seiner äußeren Mantelfläche 281 zumindest eine in Rotationsrichtung des Sperrelements 279 umlaufende Sperrlippe 283 aufweisen. Um einen Fluidstrom 14 durch den Verbindungskanalbereich 220 zu unterbinden oder zumindest zu begrenzen, kann das Sperrelement 279 zumindest bereichsweise gequetscht werden, um die Querschnittsfläche des Durchflusskanals (hier nicht sichtbar) durch das Sperrelement 279 zu verringern oder den Durchflusskanal zumindest bereichsweise vollständig zu schließen, so dass ein Durchfluss vollständig unterbunden wird. Der Vorgang des Quetschens kann mechanisch über einen Stößel 285 erfolgen, welcher, wie in Figur 12b) gezeigt, translatorisch zumindest bereichsweise in der Adapterplatte 200 gelagert ist. Durch eine Vorwärtsbewegung kann der Stößel 285 das Sperrelement 279 quetschen. Durch eine Rückwärtsbewegung kann der Stößel 285 den Durchflusskanal wieder erweitern oder vollständig freigeben. Vorzugsweise ist das Sperrelement 279 derart in dem Verbindungskanalbereich 220 angeordnet, dass die äußere Mantelfläche 281 des Sperrelements 279 fluiddicht mit dem Verbindungskanalbereich 220 abschließt. Hierzu trägt vorzugsweise die an der äußeren Mantelfläche 281 des Sperrelements 279 angeordnete eine Sperrlippe 283 bei. Um zu vermeiden, dass in einer gequetschten Position des Sperrelements 279 kein Fluid zwischen dem Sperrelement 279 und dem Verbindungskanalbereich 220 strömen kann, wird in der gequetschten Position des Sperrelements 279 vorzugsweise nur ein mittlerer Teilbereich gequetscht.

Alternativ zu einem Quetschventil kann der Flussbegrenzer 278 mittels zumindest zwei kreuzförmiger Plättchen ausgebildet sein, die drehbar in dem Verbindungskanalbereich 220 angeordnet sind. Die kreuzförmigen Plättchen weisen eine gemeinsame Rotationsachse auf, die der Strömungsrichtung des Fluidstroms 14 entspricht. Vorzugsweise liegen die Plättchen aufeinander. In einer ersten Position der Plättchen sind die Kreuzarme derart angeordnet, dass ein Durchfluss des Fluidstroms 14 zwischen den Kreuzarmen ermöglicht wird. In einer zweiten Position der Plättchen können die Kreuzarme derart angeordnet werden, dass ein Durchfluss verringert wird oder vollständig unterbunden wird. Die Plättchen können als Edelstahl-Federplättchen ausgebildet sein. Eine Druckdifferenz beeinflusst hierbei die Position der Plättchen zueinander. Ein geringer werdender Differenzdruck bewirkt eine Vergrößerung der Öffnung, eine Druckerhöhung dementsprechend eine Verkleinerung. Die durch die Adapterplatte 200 strömende Fluidmenge kann hierdurch konstant gehalten werden.

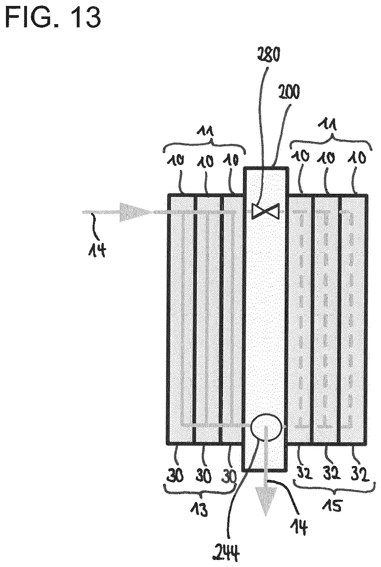

**Figur 13** zeigt ein weiteres Prozessiersystem 100 mit einer ersten und zweiten Prozessiereinheitengruppe 13 und 15. Eine Adapterplatte 200, welche in das Prozessiersystem 100 integriert ist, wird dazu genutzt, zumindest eine weitere Prozessiereinheit 10 hinzuzuschalten, um die Filterfläche bei Bedarf zu erweitern. Wie in Figur 13 gezeigt, kann durch die Adapterplatte 200 die zweite Prozessiereinheitengruppe 15 hinzugeschaltet werden. Es ist jedoch auch möglich, lediglich eine zweite Prozessiereinheit 32 hinzuzuschalten.

Wie in Figur 13 gezeigt, ist in bzw. an die Adapterplatte 200 vorzugsweise zumindest ein Erweiterungsventil 280 integriert bzw. vorgesehen. Dieses Erweiterungsventil 280 ist vorzugsweise in bzw. an dem zweiten Kanalbereich 218 angeordnet. Da die erste und zweite Prozessiereinheitengruppe 13 und 15, welche durch die Adapterplatte 200 gekoppelt sind, hier parallel verschaltet sind, kann mit Hilfe des Erweiterungsventils 280 ein Fluidstrom 14 von dem Zulaufkanal 24 der ersten Prozessiereinheit 30, die direkt mit der Adapterplatte 200 gekoppelt ist, zu dem Zulaufkanal 24 der zweiten Prozessiereinheit 32, die direkt mit der Adapterplatte 200 gekoppelt ist, ermöglicht werden, sollte eine Erweiterung der Filterfläche gewünscht sein. Das Erweiterungsventil 280 kann als selbstregulierendes Ventil, wie z.B. ein Überdruckventil, oder als ein extern gesteuertes Ventil ausgebildet sein.

Weiterhin kann in der Adapterplatte 200 zumindest ein Hilfsausgang 244 ausgebildet sein, der vorzugsweise in bzw. an dem ersten Kanalbereich 216 des Adapterkanals 214 angeordnet ist. Über diesen Hilfsausgang 244 kann, sollten die erste und zweite Prozessiereinheitengruppe 13 und 15 nicht miteinander gekoppelt sein (d.h. keine Filtererweiterung), der Fluidstrom 14 aus dem Ablaufkanal 26 der ersten Prozessiereinheit 30, die direkt mit der Adapterplatte 200 gekoppelt ist, aus dem Prozessiersystem 100 abfließen. Sind die erste und zweite Prozessiereinheitengruppe 13 und 15 jedoch miteinander gekoppelt, d.h. es findet eine Filtererweiterung statt, kann über den Hilfsausgang 244 der Adapterplatte 200 außerdem der Fluidstrom 14 aus zumindest dem Ablaufkanal 26 der zweiten Prozessiereinheit 32, die direkt mit der Adapterplatte 200 gekoppelt ist, abfließen. Es wird diesbezüglich darauf hingewiesen, dass obwohl in der übrigen Beschreibung die erste Austrittsöffnung 210 derart beschrieben wird, dass hier ein Fluid aus der Adapterplatte 200 in den Ablaufkanal 26 der zweiten Prozessiereinheit 32 ausströmen kann, die erste Austrittsöffnung 210 nicht ausschließlich für diese eine Strömungsrichtung ausgelenkt zu sein hat. Die erste Austrittsöffnung 210 kann, wie beispielsweise in Figur 13, auch dazu genutzt werden, dass ein Fluid (hier Filtrat) aus dem Ablaufkanal 26 der zweiten Prozessiereinheit 32, die direkt mit der Adapterplatte 200 gekoppelt ist, zurück in die Adapterplatte 200 strömen kann, um dann anschließend über den Hilfsausgang 244 aus der Adapterplatte 200 ausströmen zu können. Um eine derartige Strömungsrichtung zu ermöglichen, kann vorzugsweise ein Rückschlagventil an der ersten Austrittsöffnung 210 angeordnet sein.

Bei der Zellabtrennung mittels Tiefenfilter variiert die filtrierbare Menge an Lösung bis zum Erreichen der Filtrationskapazität durch unterschiedliche Zelltypen, Zelldichten und Filtereigenschaften. Mittels einer oben beschriebenen Adapterplatte 200 kann hier die Filterfläche bei Bedarf entsprechend erweitert werden. Damit muss der Filtrationsvorgang nicht unterbrochen werden. Weiterhin könnten nicht verwendete Prozessiereinheiten 10 in einer weiteren Filtration eingesetzt werden.

Es wird darauf hingewiesen, dass die Ausführungsform der Figur 13 lediglich in Bezug auf eine parallele Verschaltung der ersten und zweiten Prozessiereinheitengruppe 13 und 15 beschrieben wurde. Es ist jedoch ebenfalls denkbar, dass die zweite Prozessiereinheitengruppe 15 seriell mit der ersten Prozessiereinheitengruppe 13 verschaltet ist. Hier könnte dann das Erweiterungsventil 280 in jedem anderen Bereich des Adapterkanals 214 angeordnet sein.

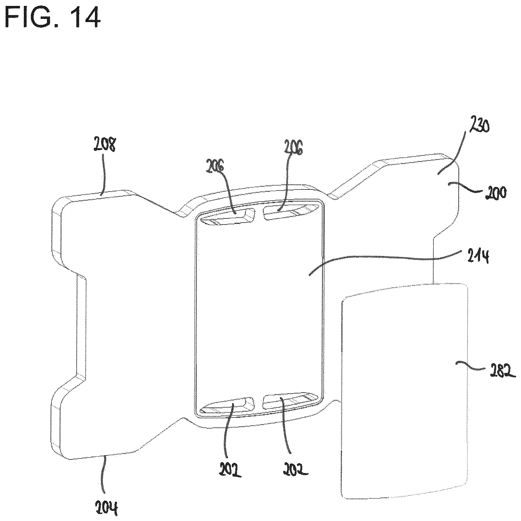

**Figur 14** zeigt eine Schnittansicht durch eine Adapterplatte 200 gemäß einer weiteren Ausführungsform. In diese Adapterplatte 200 ist zumindest eine Berstdruckscheibe 282 integriert bzw. vorgesehen, die zumindest bereichsweise in dem Bereich angeordnet ist, in dem sich der Adapterkanal 214 befindet. Die Berstdruckscheibe 282 kann aus Edelstahl, Graphit, Silikon oder Kunststoff gefertigt sein. Die Berstdruckscheibe 282 ist vorteilhafterweise dann in der Adapterplatte 200 vorzusehen, wenn mit hohen Drücken (z.B. bei Drücken zwischen 1 bis 5 bar) in der Adapterplatte 200 zu rechnen ist. In einer zweiteiligen Adapterplatte 200, welche eine Zulaufplatte 230 und eine Ablaufplatte 232 aufweist, ist die Berstdruckscheibe 282 zwischen diesen Platten angeordnet, so dass ein Fluidstrom 14 durch die Adapterplatte 200 blockiert wird. Verblockt jedoch beispielsweise das Filtermedium 16 der ersten Prozessiereinheit 30, steigt der Druck und die Berstdruckscheibe 282 bricht, so dass ein Fluidstrom 14 durch die Adapterplatte 200 zu der zweiten Prozessiereinheit 32 gewährt ist.

Weitere Anmerkungen zu den vorangehend beschriebenen Ausführungsformen:
In verschiedenen Ausführungsformen wird die Adapterplatte 200 zur Verknüpfung zweier Prozessiereinheitengruppen 11 beschrieben. Es wird jedoch darauf hingewiesen, dass die Beschreibungen ebenfalls entsprechend für Ausführungsformen gelten, in denen nur eine erste und/oder zweite Prozessiereinheit 30 und 32 vorgesehen sind.

In den beschriebenen Prozessiersystemen 100 wird jeweils eine Adapterplatte 200 gezeigt, die zwei Prozessiereinheitengruppen 11 koppelt. Es ist jedoch möglich, dass in einem Prozessiersystem 100 eine Vielzahl von Adapterplatten 200 enthalten ist. Prozessiereinheitengruppen 11, die durch eine Adapterplatte 200 gekoppelt sind, werden hier immer als erste und zweite Prozessiereinheitengruppe 13 und 15 benannt und die obige Beschreibung gilt entsprechend für jede Verknüpfung zweier Prozessiereinheitengruppen 11. Gleiches gilt ebenfalls für einzelne durch eine Adapterplatte 200 verknüpfte Prozessiereinheiten 10 oder einzelne Prozessiereinheiten 10, die mit einer Prozessiereinheitengruppe 11 verknüpft sind. Weiterhin wird darauf hingewiesen, dass, obwohl die gezeigten Adapterplatten 200 zweiteilig ausgebildet sind, es ebenfalls denkbar ist, diese einteilig auszubilden.

In der obigen Beschreibung wird auf den biopharmazeutischen Einsatz der Prozessiersysteme 100 bzw. der Adapterplatten 200 abgezielt. Es ist jedoch ebenfalls möglich, das gezeigte Prinzip auf andere Prozesse zu übertragen, wie z.B. die Lebensmittelherstellung, chemische Produktionsprozesse, Getränkefiltration, Fraktionieren von Partikeln, Schmutzwasseraufbereitung und dgl.

Insbesondere wird darauf hingewiesen, dass die Adapterplatte 200 in den beschriebenen Ausführungsformen jeweils direkt bzw. ohne Zwischenschaltung weiterer Elemente mit einer zweiten Prozessiereinheit 32 gekoppelt ist. Es ist jedoch möglich, die Adapterplatte 200 mit der zweiten Prozessiereinheit 32 über eine kompatible Konnektierung zu verbinden. Diese ist vorzugsweise steril und/oder tropffrei ausgebildet.

Die Adapterplatte 200 kann außerdem auch vor und/oder nach der letzten Prozessiereinheit 10 eines Prozessiersystems 100 angeordnet sein. Als eine oder mehrere Prozessiereinheiten 10 kann insbesondere eine Filterkassette "Sartoclear^{®} Depth Filter Cassette" und/oder eine Filterkassette der "Sartoclear^{®} DL-Serie" und/oder eine Filterkassette der "Sartoclear^{®} S-Serie" von Sartorius Stedim Biotech GmbH zum Einsatz kommen.

Die verschiedenen Ausführungsformen der Prozessiersysteme 100 bzw. der Adapterplatten 200 wurden getrennt anhand der einzelnen Figuren beschrieben. Es wird jedoch darauf hingewiesen, dass die einzelnen Ausführungsformen bzw. Teile der einzelnen Ausführungsformen miteinander kombinierbar sind. Um Wiederholungen zu vermeiden, wurden außerdem bezüglich der einzelnen Ausführungsformen bereits beschriebene Elemente nicht noch einmal beschrieben.

Die vorliegende Anmeldung kann sich weiterhin auf eine oder mehrere der folgenden Items beziehen:
Item 1. Modulares Prozessiersystem (100) für biopharmazeutische Prozesse umfassend:
   - zumindest eine erste und zweite Prozessiereinheit (30, 32), welche fluidisch miteinander verbindbar sind;
   - zumindest eine Adapterplatte (200), welche von zumindest einem Fluidstrom (14) durchströmbar ist, welcher von der ersten Prozessiereinheit (30) zu der zweiten Prozessiereinheit (32) strömt;
   wobei die Adapterplatte (200) derart ausgebildet ist, dass der Fluidstrom (14) zwischen der ersten Prozessiereinheit (30) und der zweiten Prozessiereinheit (32) mit Hilfe der Adapterplatte (200) zumindest teilweise umlenkbar ist und/oder der Fluidstrom (14), bevorzugt dessen Druck, mit Hilfe der Adapterplatte (200) regel- oder steuerbar ist.
Item 2. Modulares Prozessiersystem (100) nach Item 1 umfassend mehrere parallel verschaltete erste Prozessiereinheiten (30) und/oder mehrere parallel verschaltete zweite Prozessiereinheiten (32), wobei die ersten Prozessiereinheiten (30) und die zweiten Prozessiereinheiten (32) jeweils eine Prozessiereinheitengruppe (11) bilden.
Item 3. Modulares Prozessiersystem (100) nach Item 1 oder 2, wobei die Adapterplatte (200) einen Adapterkanal (214) aufweist, durch den der Fluidstrom (14) strömen kann, wobei der Fluidstrom (14) über zumindest eine Zutrittsöffnung in den Adapterkanal (214) einströmbar ist und über zumindest eine Austrittsöffnung ausströmbar ist.
Item 4. Modulares Prozessiersystem (100) nach Item 3, wobei die zumindest eine Zutrittsöffnung derart angeordnet ist, dass diese mit einem Ablaufkanal (26) der ersten Prozessiereinheit (30) verbindbar ist und/oder
   wobei die zumindest eine Austrittsöffnung derart angeordnet ist, dass diese mit einem Zulaufkanal (24) der zweiten Prozessiereinheit (32) verbindbar ist.
Item 5. Modulares Prozessiersystem (100) nach Item 3 oder 4, wobei die Adapterplatte (200) zumindest zwei Zutrittsöffnungen und zumindest zwei Austrittsöffnungen aufweist,
   wobei zumindest eine erste Zutrittsöffnung (202) derart angeordnet ist, dass der Fluidstrom (14) aus einem Ablaufkanal (26) der ersten Prozessiereinheit (30) in den Adapterkanal (214) einströmbar ist,
   wobei zumindest eine zweite Zutrittsöffnung (206) derart angeordnet ist, dass der Fluidstrom (14) aus einem Zulaufkanal (24) der ersten Prozessiereinheit (30) in den Adapterkanal (214) einströmbar ist,
   wobei zumindest eine erste Austrittsöffnung (210) derart angeordnet ist, dass der Fluidstrom (14) aus der ersten Austrittsöffnung (210) in einen Ablaufkanal (26) der zweiten Prozessiereinheit (32) einströmbar ist, und
   wobei zumindest eine zweite Austrittsöffnung (212) derart angeordnet ist, dass der Fluidstrom (14) aus der zweiten Austrittsöffnung (212) in einen Zulaufkanal (24) der zweiten Prozessiereinheit (32) einströmbar ist.
Item 6. Modulares Prozessiersystem (100) nach Item 5, wobei der Adapterkanal (214) umfasst:
   - einen ersten Kanalbereich (216), der zwischen der ersten Zutrittsöffnung (202) und der ersten Austrittsöffnung (210) angeordnet ist;
   - einen zweiten Kanalbereich (218), der zwischen der zweiten Zutrittsöffnung (206) und der zweiten Austrittsöffnung (212) angeordnet ist; und
   - einen Verbindungskanalbereich (220), der den ersten und zweiten Kanalbereich (216, 218) fluidisch verbindet.
Item 7. Modulares Prozessiersystem (100) nach Item 5 oder 6, wobei in dem Adapterkanal (214) zumindest ein Umlenkelement derart verschiebbar angeordnet ist, dass ein Fluidstrom (14) durch die Adapterplatte (200) derart umgelenkt werden kann, dass entweder:
   - ein erster Fluidstrom über die erste Zutrittsöffnung (202) in den Adapterkanal (214) einströmen kann und über die erste Austrittsöffnung (210) ausströmen kann und ein zweiter Fluidstrom über die zweite Zutrittsöffnung (206) in den Adapterkanal (214) einströmen kann und über die zweite Austrittsöffnung (212) aus dem Adapterkanal (214) ausströmen kann;
   oder:
   - ein Fluidstrom (14) über die erste Zutrittsöffnung (202) in den Adapterkanal (214) einströmen kann und über die zweite Austrittsöffnung (212) aus dem Adapterkanal (214) ausströmen kann.
Item 8. Modulares Prozessiersystem (100) nach Item 6, umfassend zwei Umlenkelemente, welche als Mehrwegventil (246) ausgebildet sind, wobei ein erstes Ventil (224) in dem ersten Kanalbereich (216) angeordnet ist und ein zweites Ventil (226) in dem zweiten Kanalbereich (218) angeordnet ist, wobei die Ventile (224, 226) derart ausgelegt sind, dass entweder:
   - ein erster Fluidstrom über die erste Zutrittsöffnung (202) in den Adapterkanal (214) einströmen kann und über die erste Austrittsöffnung (210) ausströmen kann und ein zweiter Fluidstrom über die zweite Zutrittsöffnung (206) in den Adapterkanal (214) einströmen kann und über die zweite Austrittsöffnung (212) aus dem Adapterkanal (214) ausströmen kann;
   oder:
   - ein Fluidstrom (14) über die erste Zutrittsöffnung (202) in den Adapterkanal (214) einströmen kann und über die zweite Austrittsöffnung (212) aus dem Adapterkanal (214) ausströmen kann.
Item 9. Modulares Prozessiersystem (100) nach Item 8, wobei das Mehrwegventil (246) ein Ventilrohr (248) umfasst, welches jeweils verlagerbar in dem ersten und zweiten Kanalbereich (216, 218) angeordnet ist und in entsprechender Weise von dem ersten und zweiten Fluidstrom durchströmbar ist,
   wobei in einer Mantelfläche (250) des Ventilrohrs (248) zumindest zwei Ventilöffnungen (252) angeordnet sind, die in Verlagerungsrichtung voneinander versetzt angeordnet sind, so dass in Abhängigkeit der Verlagerungsstellung des Ventilrohrs (248) in dem jeweiligen Kanalbereich ein Fluidstrom (14) durch den Adapterkanal (214) umlenkbar ist.
Item 10. Modulares Prozessiersystem (100) nach einem der Items 3 bis 9, wobei in bzw. an dem Adapterkanal (214) zumindest ein Sensor (228) angeordnet ist.
Item 11. Modulares Prozessiersystem (100) nach einem der Items 3 bis 10, wobei der Adapterkanal (214) mit zumindest einem Hilfszu- und/oder Hilfsausgang (244) ausgebildet ist.
Item 12. Modulares Prozessiersystem (100) nach einem der Items 3 bis 11, wobei in bzw. an dem Adapterkanal (214) zumindest eine Pumpe (258) angeordnet ist.
Item 13. Modulares Prozessiersystem (100) nach Item 12, wobei die Pumpe (258) als Peristaltikpumpe oder Kolbenpumpe ausgebildet ist.
Item 14. Modulares Prozessiersystem (100) nach einem der Items 3 bis 13, wobei in dem Adapterkanal (214) zumindest ein Flussbegrenzer (278) angeordnet ist.
Item 15. Verfahren zum modularen Aufbau eines Prozessiersystems (100) für biopharmazeutische Prozesse umfassend die Schritte:
   - Bereitstellen zumindest einer ersten und zweiten Prozessiereinheit (30, 32);
   - Bereitstellen zumindest einer Adapterplatte (200), welche dazu ausgelegt ist, die erste und zweite Prozessiereinheit (30, 32) fluidisch zu verbinden, so dass zumindest ein Fluidstrom (14) von der ersten Prozessiereinheit (30) zu der zweiten Prozessiereinheit (32) durch die Adapterplatte (200) strömen kann; und
   - Verbinden der ersten und zweiten Prozessiereinheit (30, 32) mit der Adapterplatte (200),
   wobei die Adapterplatte (200) derart gewählt und/oder eingestellt wird, dass der Fluidstrom (14) zwischen der ersten Prozessiereinheit (30) und der zweiten Prozessiereinheit (32) mit Hilfe der Adapterplatte (200) in vordefinierter Weise zumindest teilweise umlenkbar ist und/oder der Fluidstrom (14), bevorzugt dessen Druck, mit Hilfe der Adapterplatte (200) in vordefinierter Weise regel- oder steuerbar ist.

### Bezugszeichenliste

- 10: Prozessiereinheit
- 11: Prozessiereinheitengruppe
- 12: Prozessiergehäuse
- 13: Erste Prozessiereinheitengruppe
- 14: Fluidstrom
- 15: Zweite Prozessiereinheitengruppe
- 16: Filtermedium
- 17: Filterträger
- 18: Filtratseite
- 19: Leerraum
- 20: Retentatseite
- 21: Schüttgut
- 22: Oberes Ende des Prozessiergehäuses
- 24: Zulaufkanal
- 26: Ablaufkanal
- 27: Zweiter Ablaufkanal
- 28: Unteres Ende des Prozessiergehäuses
- 30: Erste Prozessiereinheit
- 32: Zweite Prozessiereinheit
- 34: Abschlusshalterung
- 36: Zuflusspumpe
- 38: Mischtank

- 100: Prozessiersystem

- 200: Adapterplatte
- 202: Erste Zutrittsöffnung
- 204: Unteres Ende der Adapterplatte
- 206: Zweite Zutrittsöffnung
- 208: Oberes Ende der Adapterplatte
- 210: Erste Austrittsöffnung
- 212: Zweite Austrittsöffnung
- 214: Adapterkanal
- 216: Erster Kanalbereich
- 218: Zweiter Kanalbereich
- 220: Verbindungskanalbereich
- 222: Ventil
- 224: Erstes Ventil
- 226: Zweites Ventil
- 228: Sensor
- 230: Zulaufplatte
- 232: Ablaufplatte
- 234: Kanalvertiefung
- 236: Strömungssteg
- 238: Verbindungskanal zum Sensor
- 240: Hilfsabzweigung
- 242: Kanalelement
- 244: Hilfsaus- bzw. -zugang
- 245: Hilfsventil
- 246: Mehrwegventil
- 248: Ventilrohr
- 250: Mantelfläche des Ventilrohrs
- 252: Ventilöffnung
- 254: Handstück
- 256: Verschlusselement
- 258: Pumpe
- 260: Kolben
- 262: Zylinder
- 264: Einlassventil
- 266: Auslassventil
- 268: Schlauch
- 270: Rotor
- 272: Oberseite der Rotorplatte
- 274: Rolle des Rotors
- 276: Zahnradmechanismus
- 278: Flussbegrenzer
- 279: Flexibles Sperrelement
- 280: Erweiterungsventil
- 281: Äußere Mantelfläche
- 282: Berstdruckscheibe
- 283: Sperrlippe
- 285: Stößel

- VR: Vertikale Richtung
- HR: Horizontale Richtung

## Patentansprüche

1. Modulares Prozessiersystem (100) für biopharmazeutische Prozesse umfassend:
- zumindest eine erste und zweite Prozessiereinheit (30, 32), welche fluidisch miteinander verbindbar sind; und
- zumindest eine Adapterplatte (200), welche von zumindest einem Fluidstrom (14) durchströmbar ist, welcher von der ersten Prozessiereinheit (30) zu der zweiten Prozessiereinheit (32) strömt;
wobei die Adapterplatte (200) einen Adapterkanal (214) aufweist, durch den der Fluidstrom (14) strömen kann, wobei der Fluidstrom (14) über zumindest eine Zutrittsöffnung in den Adapterkanal (214) einströmbar ist und über zumindest eine Austrittsöffnung ausströmbar ist;
wobei der Adapterkanal (214) mit zumindest einem Hilfszu- und/oder Hilfsausgang (244) ausgebildet ist; und
wobei in dem Adapterkanal (214) zumindest ein Ventil angeordnet ist, welches dazu ausgelegt ist, den Fluidstrom (14) zwischen der ersten Prozessiereinheit (30) und der zweiten Prozessiereinheit (32) zumindest teilweise umzulenken.

2. Modulares Prozessiersystem (100) nach Anspruch 1, wobei mit Hilfe des zumindest einen Hilfszu- und/oder Hilfsausgangs (244) der Fluidstrom, der durch die Adapterplatte (200) fließt, zumindest teilweise aus der Adapterplatte (200) ab- und/oder wieder zugeführt wird.

3. Modulares Prozessiersystem (100) für biopharmazeutische Prozesse umfassend:
- zumindest eine erste und zweite Prozessiereinheit (30, 32), welche fluidisch miteinander verbindbar sind; und
- zumindest eine Adapterplatte (200), welche von zumindest einem Fluidstrom (14) durchströmbar ist, welcher von der ersten Prozessiereinheit (30) zu der zweiten Prozessiereinheit (32) strömt;
wobei die Adapterplatte (200) einen Adapterkanal (214) aufweist, durch den der Fluidstrom (14) strömen kann, wobei der Fluidstrom (14) über zumindest eine Zutrittsöffnung in den Adapterkanal (214) einströmbar ist und über zumindest eine Austrittsöffnung ausströmbar ist;
wobei in bzw. an dem Adapterkanal (214) zumindest ein Sensor (228) zur Messung des Volumenstroms, des UV-Werts, des pH-Werts, der Trübung und/oder der Viskosität des Fluids angeordnet ist; und
wobei in dem Adapterkanal (214) zumindest ein Ventil angeordnet ist, welches dazu ausgelegt ist, den Fluidstrom (14) zwischen der ersten Prozessiereinheit (30) und der zweiten Prozessiereinheit (32) zumindest teilweise umzulenken.

4. Modulares Prozessiersystem (100) nach Anspruch 3, umfassend ein externes Steuergerät, an welches die gemessenen Werte des zumindest einen Sensors (228) übermittelt werden, wobei das externe Steuergerät dazu ausgelegt ist, die gemessenen Werte zum Regeln und/oder Steuern des Prozesses heranzuziehen und/oder durch welches das zumindest eine Ventil gesteuert ist.

5. Modulares Prozessiersystem (100) nach einem der Ansprüche 1-4, umfassend mehrere parallel verschaltete erste Prozessiereinheiten (30) und/oder mehrere parallel verschaltete zweite Prozessiereinheiten (32), wobei die ersten Prozessiereinheiten (30) und die zweiten Prozessiereinheiten (32) jeweils eine Prozessiereinheitengruppe (11) bilden.

6. Modulares Prozessiersystem (100) nach einem der vorangehenden Ansprüche, wobei die zumindest eine Zutrittsöffnung derart angeordnet ist, dass diese mit einem Ablaufkanal (26) der ersten Prozessiereinheit (30) verbindbar ist und/oder
wobei die zumindest eine Austrittsöffnung derart angeordnet ist, dass diese mit einem Zulaufkanal (24) der zweiten Prozessiereinheit (32) verbindbar ist.

7. Modulares Prozessiersystem (100) nach einem der vorangehenden Ansprüche, wobei die Adapterplatte (200) zumindest zwei Zutrittsöffnungen und zumindest zwei Austrittsöffnungen aufweist,
wobei zumindest eine erste Zutrittsöffnung (202) derart angeordnet ist, dass der Fluidstrom (14) aus einem Ablaufkanal (26) der ersten Prozessiereinheit (30) in den Adapterkanal (214) einströmbar ist,
wobei zumindest eine zweite Zutrittsöffnung (206) derart angeordnet ist, dass der Fluidstrom (14) aus einem Zulaufkanal (24) der ersten Prozessiereinheit (30) in den Adapterkanal (214) einströmbar ist,
wobei zumindest eine erste Austrittsöffnung (210) derart angeordnet ist, dass der Fluidstrom (14) aus der ersten Austrittsöffnung (210) in einen Ablaufkanal (26) der zweiten Prozessiereinheit (32) einströmbar ist, und
wobei zumindest eine zweite Austrittsöffnung (212) derart angeordnet ist, dass der Fluidstrom (14) aus der zweiten Austrittsöffnung (212) in einen Zulaufkanal (24) der zweiten Prozessiereinheit (32) einströmbar ist.

8. Modulares Prozessiersystem (100) nach Anspruch 7, wobei der Adapterkanal (214) umfasst:
- einen ersten Kanalbereich (216), der zwischen der ersten Zutrittsöffnung (202) und der ersten Austrittsöffnung (210) angeordnet ist;
- einen zweiten Kanalbereich (218), der zwischen der zweiten Zutrittsöffnung (206) und der zweiten Austrittsöffnung (212) angeordnet ist; und
- einen Verbindungskanalbereich (220), der den ersten und zweiten Kanalbereich (216, 218) fluidisch verbindet.

9. Modulares Prozessiersystem (100) nach Anspruch 7 oder 8, wobei in dem Adapterkanal (214) zumindest ein Umlenkelement derart verschiebbar angeordnet ist, dass ein Fluidstrom (14) durch die Adapterplatte (200) derart umgelenkt werden kann, dass entweder:
- ein erster Fluidstrom über die erste Zutrittsöffnung (202) in den Adapterkanal (214) einströmen kann und über die erste Austrittsöffnung (210) ausströmen kann und ein zweiter Fluidstrom über die zweite Zutrittsöffnung (206) in den Adapterkanal (214) einströmen kann und über die zweite Austrittsöffnung (212) aus dem Adapterkanal (214) ausströmen kann;
oder:
- ein Fluidstrom (14) über die erste Zutrittsöffnung (202) in den Adapterkanal (214) einströmen kann und über die zweite Austrittsöffnung (212) aus dem Adapterkanal (214) ausströmen kann.

10. Modulares Prozessiersystem (100) nach Anspruch 8, umfassend zwei Umlenkelemente, welche als Mehrwegventil (246) ausgebildet sind, wobei ein erstes Ventil (224) in dem ersten Kanalbereich (216) angeordnet ist und ein zweites Ventil (226) in dem zweiten Kanalbereich (218) angeordnet ist, wobei die Ventile (224, 226) derart ausgelegt sind, dass entweder:
- ein erster Fluidstrom über die erste Zutrittsöffnung (202) in den Adapterkanal (214) einströmen kann und über die erste Austrittsöffnung (210) ausströmen kann und ein zweiter Fluidstrom über die zweite Zutrittsöffnung (206) in den Adapterkanal (214) einströmen kann und über die zweite Austrittsöffnung (212) aus dem Adapterkanal (214) ausströmen kann;
oder:
- ein Fluidstrom (14) über die erste Zutrittsöffnung (202) in den Adapterkanal (214) einströmen kann und über die zweite Austrittsöffnung (212) aus dem Adapterkanal (214) ausströmen kann.

11. Modulares Prozessiersystem (100) nach Anspruch 10, wobei das Mehrwegventil (246) ein Ventilrohr (248) umfasst, welches jeweils verlagerbar in dem ersten und zweiten Kanalbereich (216, 218) angeordnet ist und in entsprechender Weise von dem ersten und zweiten Fluidstrom durchströmbar ist,
wobei in einer Mantelfläche (250) des Ventilrohrs (248) zumindest zwei Ventilöffnungen (252) angeordnet sind, die in Verlagerungsrichtung voneinander versetzt angeordnet sind, so dass in Abhängigkeit der Verlagerungsstellung des Ventilrohrs (248) in dem jeweiligen Kanalbereich ein Fluidstrom (14) durch den Adapterkanal (214) umlenkbar ist.

12. Modulares Prozessiersystem (100) nach einem der vorangehenden Ansprüche, wobei in bzw. an dem Adapterkanal (214) zumindest eine Pumpe (258), vorzugsweise eine Peristaltikpumpe oder Kolbenpumpe, angeordnet ist.

13. Modulares Prozessiersystem (100) nach einem der Ansprüche 1 oder 2, wobei über den zumindest einem Hilfszu- und/oder Hilfsausgang (244) eine Pumpe extern zugeschaltet ist.

14. Verfahren zum modularen Aufbau eines Prozessiersystems (100) für biopharmazeutische Prozesse umfassend die Schritte:
- Bereitstellen zumindest einer ersten und zweiten Prozessiereinheit (30, 32);
- Bereitstellen zumindest einer Adapterplatte (200), welche dazu ausgelegt ist, die erste und zweite Prozessiereinheit (30, 32) fluidisch zu verbinden, so dass zumindest ein Fluidstrom (14) von der ersten Prozessiereinheit (30) zu der zweiten Prozessiereinheit (32) durch die Adapterplatte (200) strömen kann; und
- Verbinden der ersten und zweiten Prozessiereinheit (30, 32) mit der Adapterplatte (200),
wobei die Adapterplatte (200) einen Adapterkanal (214) aufweist, durch den der Fluidstrom (14) strömen kann, wobei der Fluidstrom (14) über zumindest eine Zutrittsöffnung in den Adapterkanal (214) einströmbar ist und über zumindest eine Austrittsöffnung ausströmbar ist;
wobei die Adapterplatte (200) derart bereitgestellt wird, dass der Adapterkanal (214) mit zumindest einem Hilfszu- und/oder Hilfsausgang (244) ausgebildet ist; und
wobei die Adapterplatte (200) derart bereitgestellt wird, dass in bzw. an dem Adapterkanal (214) zumindest ein Ventil angeordnet ist, welches dazu ausgelegt ist, den Fluidstrom (14) zwischen der ersten Prozessiereinheit (30) und der zweiten Prozessiereinheit (32) zumindest teilweise umzulenken.

15. Verfahren zum modularen Aufbau eines Prozessiersystems (100) für biopharmazeutische Prozesse umfassend die Schritte:
- Bereitstellen zumindest einer ersten und zweiten Prozessiereinheit (30, 32);
- Bereitstellen zumindest einer Adapterplatte (200), welche dazu ausgelegt ist, die erste und zweite Prozessiereinheit (30, 32) fluidisch zu verbinden, so dass zumindest ein Fluidstrom (14) von der ersten Prozessiereinheit (30) zu der zweiten Prozessiereinheit (32) durch die Adapterplatte (200) strömen kann; und
- Verbinden der ersten und zweiten Prozessiereinheit (30, 32) mit der Adapterplatte (200),
wobei die Adapterplatte (200) einen Adapterkanal (214) aufweist, durch den der Fluidstrom (14) strömen kann, wobei der Fluidstrom (14) über zumindest eine Zutrittsöffnung in den Adapterkanal (214) einströmbar ist und über zumindest eine Austrittsöffnung ausströmbar ist;
wobei die Adapterplatte (200) derart bereitgestellt wird, dass in bzw. an dem Adapterkanal (214) zumindest ein Sensor (228) angeordnet ist; und
wobei die Adapterplatte (200) derart bereitgestellt wird, dass in bzw. an dem Adapterkanal (214) zumindest ein Ventil angeordnet ist, welches dazu ausgelegt ist, den Fluidstrom (14) zwischen der ersten Prozessiereinheit (30) und der zweiten Prozessiereinheit (32) zumindest teilweise umzulenken.

16. Verwendung eines modularen Prozessiersystems (100) gemäß Anspruch 1 zur Durchführung eines biopharmazeutischen Prozesses, wobei über den zumindest eine Hilfszu- und/oder Hilfsausgang (244):
- ein Integritätstest an unterschiedlichen Prozessiereinheiten innerhalb des Prozessiersystems vorgenommen wird,
- eine erste Prozessiereinheit getrennt gereinigt wird,
- zum Lösungsmittelaustausch ein Diafiltrationsmedium zugeführt wird,
- durch Medienzugabe eine Elution/Waschen/Reinigen einer vor- oder nachgeschalteten Chromatographiefiltervorrichtung erfolgt,
- das Prozessiersystem entlüftet wird, oder
- Proben entnommen werden.

17. Verwendung des modularen Prozessiersystems (100) gemäß Anspruch 2
a) zur externen Verarbeitung des abgezweigten Fluidstroms aus mindestens einem vorgelagerten Prozessierschritt bei einer (kontinuierlichen) Virusinaktivierung,
b) zum zwischenzeitlichen Puffern und/oder Regenerieren des abgeführten Fluidstroms aus der ersten Prozessiereinheit.
